# NEUE EUROPÄISCHE PATENTSCHRIFT

(11) **EP 2 662 434 B2**
(45) Veröffentlichungstag und Bekanntmachung des Hinweises auf die Entscheidung über den Einspruch: **16.08.2023**
(45) Hinweis auf die Patenterteilung: 21.03.2018
(21) Anmeldenummer: 13171453.7
(22) Anmeldetag: 10.05.2012
(51) Int. Cl.: C11B 9/00, A61Q 13/00, C11D 3/50, A61L 9/01, A61Q 19/10, A61Q 5/02, A61K 8/49, A61K 8/35

(54) **Verwendung bestimmter Verbindungen zum Verstärken von Gerüchen**
Use of specific compounds for enhancing odours
Utilisation de certaines liaisons pour augmenter des odeurs

(43) Veröffentlichungstag der Anmeldung: 13.11.2013
(62) Teilanmeldung aus: 12167516.9
(73) Patentinhaber: Symrise AG, 37603 Holzminden (DE)
(72) Erfinder: Hölscher, Bernd, 37620 Halle (DE); Chmelnyk, Annabel, 37603 Holzminden (DE)
(74) Vertreter: Fabry, Bernd

(56) Entgegenhaltungen:
- EP-A1- 1 201 738
- EP-A1- 2 578 671
- EP-A2- 0 818 452
- WO-A1-97/32948
- WO-A1-2005/063670
- WO-A1-2005/123889
- WO-A1-2012/080235
- DE-A1- 19 743 718
- US-A- 3 927 107
- US-A- 4 541 950
- US-A- 5 266 559
- US-B1- 6 225 281
- "Symrise Perfumes Compendium", Ambrettolide, Globalide®, Globanone®, Isomuscone® and Macrolide®, 2007,

## Beschreibung

Die Erfindung betrifft die Verwendung einer einzelnen Verbindung der Formel (I) oder einer Mischung umfassend oder bestehend aus zwei oder mehr Verbindungen der Formel (I) (wie hierin beschrieben) 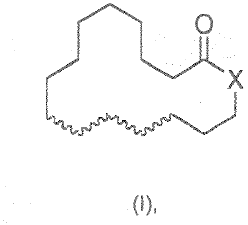 wobei für die Verbindung der Formel (I) bzw. jede Verbindung der Formel (I) gilt, dass keine, eine oder zwei der vier gewellten Linien eine Doppelbindung bedeutet bzw. bedeuten und die übrigen gewellten Linien jeweils eine Einfachbindung bedeuten und X ausgewählt ist aus -O-, -CH2- und -O-CH2-, zum Verstärken des bzw. eines oder mehrerer angenehmer Geruchseindrücke eines oder mehrerer angenehm riechender Stoffe, wobei der/die Stoff(e) keine Verbindung der Formel (I) ist bzw. sind,
wobei (i) die Verbindung der Formel (I) bzw. (ii) eine, mehrere oder sämtliche Verbindungen der Formel (I) ausgewählt ist bzw. sind aus der Gruppe bestehend aus Cyclohexadec-8-en-1-on (Aurelione, CAS No. 88642-03-9, 3100-36-5; Globanone, CAS No. 3100-36-5) und Cyclohexadecanon (Isomuscone, CAS No. 2550-52-9).
und wobei der bzw. einer, mehrere oder sämtliche der angenehm riechenden, nicht der Formel (I) entsprechende(n) Stoff(e) ausgewählt ist bzw. sind aus der Gruppe bestehend aus Methyldihydrojasmonat, Benzylsalicylat, cis-3-Hexenylsalicylat, Isoamylsalicylat, Hexylsalicylat, 2,3,8,8-Tetramethyl-1,2,3,4,5,6,7,8-octahydro-2-naphthalenylmethylketon, Linalylacetat, Ethyllinalylacetat, Cedrylmethylether, Cedrylmethylketon, Cedrylacetat, (4aR,5R,7aS,9R)-Octahydro-2,2,5,8,8,9a-hexamethyl-4H-4a,9-methano azuleno(5,6-d)-1,3-dioxol), 1',1',5',5'-Tetra methyl-hexahydro-spiro[1.3-dioxolan-2.8'(5'H)-2H-2.4a]methanonaphthalin, Cyclododecylmethylether, (Ethoxymethoxy)cyclododecan, Decahydro-beta-naphthylacetat, 4,7-Methano-3a,4,5,6,7,7a-hexahydro-5(-6)-indenylacetat, Allyl-3-cyclohexylpropionat, Allylcyclohexyloxyacetat, Benzylbenzoat, Benzylcinnamat, 15-Hydroxy-Pentadecanonsäurelacton, 3-Methyl-cyclopentadecenon, 1,3,4,6,7,8-hexahydro-4,6,6,7,8,8-hexamethyl Cyclopenta[g]-2-benzopyran, 2-[1-(3,3-dimethylcyclohexyl)ethoxy]-2-methyl-, 1-propanoat, 1,4-Dioxacycloheptadecan-5,17-dion, 3-Methycyclopentadecanon, 3a,6,6,9a-Tetramethyl dodecahydronaphtho[2,1-b]furan, alpha-Irone, beta-Irone, alpha-n-Methylionon, beta-n-Methylionon, alpha-Isomethylionon, beta-Isomethylionon und Allylionon, 2-Methyl-3-(4-tert-butylphenyl)propanal, 4-(4-Hydroxy-4-methylpentyl)-3-cyclohexencarboxaldehyd, 2-Methyl-4-(2,6,6-trimethyl-1-cyclohexen-1-yl)-2-butenal, 1-Methyl-4-(4-methyl-3-penten-1-yl)-3-cyclohexencarbox aldehyd, 3-(3-Isopropyl-phenyl)-butyraldehyd, (E)-2,6,10-Trimethyl-undeca-5,9-dienal, Benzo[1,3]dioxole-5-carbaldehyd, 2,2-Dimethyl-3-phenyl-propan-1-ol, 2,2-Dimethyl-3-m-tolyl-propan-1-ol, 1-(4-Isopropyl-cyclohexyl)-ethanol, (4-Isopropyl-cyclohexyl)-methanol, 2-Phenyl-ethanol, 2-Isobutyl-4-methyl-tetrahydro-pyran-4-ol, 3,7-Dimethyl-octa-1,6-dien-3-ol, (Z)-3,7-Dimethyl-octa-2,6-dien-1-ol, (E)-3,7-Dimethyl-octa-2,6-dien-1-ol, 3,7-Dimethyl-oct-6-en-1-ol, 2,6-Dimethyl-oct-7-en-2-ol, 3,7-Dimethyl-octan-1-ol, 2-Methyl-6-methylenoct-7-en-2-ol und (E/Z)-3,7-Dimethyl-nona-1,6-dien-3-ol.

Die hierin beschriebenen Verbindungen der Formel (I) eignen sich vorteilhafterweise auch zum Maskieren oder Vermindern des bzw. eines oder mehrerer unangenehmer Geruchseindrücke eines oder mehrerer unangenehm riechender (nicht der Formel (I) entsprechender) Stoffe.
Die Erfindung betrifft weiterhin neue Riechstoffmischungen, vorzugsweise Parfümöle, die eine oder mehrere solcher Verbindungen der Formel (I) sowie eine oder mehrere bestimmte (weitere) Riechstoffe (wie hierin beschrieben) enthalten oder daraus bestehen, wobei das Verhältnis der Gesamtmasse an nicht der Formel (I) entsprechenden Riechstoffen zur Gesamtmasse an Verbindung(en) der Formel (I) größer oder gleich 99,9 : 0,1, besonders bevorzugt größer oder gleich 99,999 : 0,001.
Ferner betrifft die vorliegende Erfindung parfümierte Produkte, die eine erfindungsgemäße Riechstoffmischung in einer sensorisch wirksamen Menge enthalten, wobei der Anteil der Riechstoffmischung bezogen auf das Gesamtgewicht des Produkts im Bereich von 0,01 bis 10 Gew.-%, bevorzugt im Bereich von 0,1 bis 5 Gew.-%, besonders bevorzugt im Bereich von 0,25 bis 3 Gew.-%, liegt.
Die vorliegende Erfindung betrifft auch ein Verfahren zum Herstellen eines parfümierten Produkts.
Weitere Aspekte der Erfindung ergeben sich aus der nachfolgenden Beschreibung, den Beispielen sowie insbesondere den beigefügten Patentansprüchen.
Grundsätzlich besteht seitens der Parfümindustrie ein ständiger Bedarf, angenehme geruchliche Aspekte von Riechstoffen zu betonen (hervorzuheben / zu verstärken) und unangenehme geruchliche Aspekte zu maskieren oder zu vermindern. Insbesondere Blumenriechstoffe, d.h. Riechstoffe mit einer blumigen Note, spielen in der Parfümerie eine wichtige Rolle. Dabei ist es gewünscht, einerseits deren natürliche Frische und/oder Ausstrahlung besonders hervorzuheben und andererseits deren fettige, metallische und/oder technische Noten zu unterdrücken.
Insbesondere die Reduzierung von unangenehmen Gerüchen ist ein parfümistischkompositorisch schwierig zu bearbeitendes und zu lösendes Problem. Vor allem die spezielle Eigenart eines jeweiligen unangenehmen Geruchs schränkt die Verwendungsmöglichkeiten stark ein. Häufig gelingt eine Reduzierung von unangenehmen Gerüchen allenfalls durch Kombinationen mit einem speziell entwickelten Parfümöl, welches selbst einen eigenen ganz bestimmten Dufttyp auf- weist. Dabei werden die unangenehmen Geruchseindrücke häufig mit Hilfe von wohlriechenden Gerüchen einer (an- deren) Substanz lediglich überdeckt.

Von erheblichem Vorteil sind daher Substanzen bzw. Stoffe, die in der Lage sind, die Intensität von unangenehmen Gerüchen zu mindern oder den unangenehmen Geruch sogar vollständig zu beseitigen, ohne selbst von einer nennenswerten geruchlichparfümistischen Intensität zu sein bzw. ohne selbst in der für die Zwecke der Geruchsreduktion einzusetzenden Konzentration von einer nennenswerten geruchlichparfümistischen Intensität zu sein. Solche (Wirk-)Stoffe könnten unangenehme Gerüche vorteilhafterweise neutralisieren, ohne den jeweiligen unangenehmen Geruch lediglich durch einen bestimmten Eigengeruch zu überdecken. Eine Verwendung solcher Stoffe hat bzw. hätte den Vorteil, dass für die Beduftung oder Parfümierung von Objekten, Zubereitungen beziehungsweise Produkten mit unangenehmen Gerüchen beispielsweise Parfümöle jeder beliebigen Duftrichtung verwendet werden können. Dadurch kann dem Verbraucher eine wesentlich breitere Auswahl an Dufttypen angeboten werden.

In der Literatur sind zahlreiche Vorschläge zur Bekämpfung von unangenehmen Gerüchen zu finden, wie beispielhaft im Folgenden dargestellt.

In WO 01/43784 und US 7,157,411 wird die Verwendung bestimmter Ester, insbesondere Isomenthylester wie Isomenthylacetat, als geruchsneutralisierende Substanzen zur Reduzierung unangenehmer Gerüche verschiedenster Art beschrieben.

Bisherige (Riech-)Stoffe oder (Riech-)Stoffmischungen, die auf eine geruchliche Aufwertung von Produkten abzielen, weisen häufig eine unzufriedenstellende Geruchsreduzierung unangenehmer Gerüche auf. Bei den im Stand der Technik bekannten Verfahren zum Reduzieren von unangenehmen Gerüchen besteht häufig der Nachteil, dass die eingesetzten (Riech-)Stoffe bzw. (Riech-)Stoffmischungen in erheblichen Mengen eingesetzt werden müssen, was zu Kosten- und Anwendungsproblemen führen kann.

Es war primäre Aufgabe der vorliegenden Erfindung, alternative oder verbesserte Stoffe oder Stoffmischungen zum Verstärken von angenehmen Gerüchen anzugeben, wobei sich diese vorzugsweise zudem dazu eignen, unangenehme Gerüche zu reduzieren, insbesondere zu maskieren und/oder zu vermindern.

Diese Substanzen sollten dabei vorzugsweise eine, mehrere oder vorzugsweise sämtliche der folgenden Anforderungen erfüllen:
- leichte Zugänglichkeit,
- hohe Wirksamkeit in geringer Konzentration, vorzugsweise bei in geringen Konzentrationen nicht oder kaum wahrnehmbarem Eigengeruch,
- weitgehende oder vollständige Farblosigkeit,
- hohe Stabilität in diversen Mischungen bzw. Zubereitungen, wobei insbesondere keine Verfärbung und/oder Separation und/oder Trübung auftreten soll,
- inertes Verhalten,
- keine toxische und/oder allergene Wirkung gegenüber dem Menschen.

Hinsichtlich des Begriffs "vermindern" ist anzumerken, dass damit eine besondere Form der Geruchsveränderung gemeint ist, nämlich die Abschwächung der Intensität eines (unangenehmen) Geruchs im Gegensatz zu einem bloßen Überlagern/Überdecken durch eine (andere) dominierende Geruchsnote. Unter einer Verminderung von (unangenehmen) Gerüchen wird im Rahmen des vorliegenden Textes eine teilweise Reduzierung oder eine vollständige Reduzierung, d.h. Beseitigung, von (unangenehmen) Gerüchen durch einen bestimmten Stoff oder eine bestimmte Stoffmischung verstanden, insbesondere von Gerüchen bzw. Geruchsnoten des Typs technisch, metallisch und/oder fettig oder von ähnlichen Gerüchen.

Des Weiteren sollten mit der vorliegenden Erfindung neue, vorteilhafte Riechstoffmischungen, insbesondere Parfümöle, angegeben werden, welche solche Substanzen enthalten. Solche Riechstoffmischungen sollten vorzugsweise dafür geeignet sein, bestimmte Produkte zu beduften beziehungsweise zu parfümieren.

Weiterhin sollten demensprechend parfümierte Produkte enthaltend eine solche Riechstoffmischungen sowie Verfahren zur Herstellung solcher Produkte angegeben werden.

Eine Parfümierung (eines Produkts) im engeren Sinne ist dabei zu unterscheiden von einer Reduzierung eines unangenehmen Geruchs (wie oben beschrieben) und kann zusätzlich zu einer solchen Reduzierung auftreten. Unter Parfümierung wird das Verleihen eines Geruchseindrucks verstanden, d.h. eines (zusätzlichen) geruchlichen Effekts.

Weitere Aufgabenstellungen, die der vorliegenden Erfindung zugrundeliegen, ergeben sich aus den nachfolgenden Ausführungen und den beigefügten Patentansprüchen.

Die primäre Aufgabe der vorliegenden Erfindung wird gelöst durch die Verwendung
(i) einer einzelnen Verbindung der Formel (I) oder
(ii) einer Mischung umfassend oder bestehend aus zwei oder mehr Verbindungen der Formel (I) 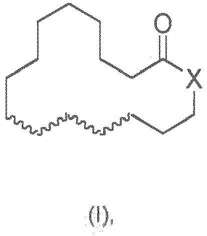 wobei für die Verbindung der Formel (I) bzw. jede Verbindung der Formel (I) gilt, dass
   keine, eine oder zwei der vier gewellten Linien eine Doppelbindung bedeutet bzw. bedeuten und die übrigen gewellten Linien jeweils eine Einfachbindung bedeuten und
   X ausgewählt ist aus -O-, -CH₂- und -O-CH₂-, vorzugsweise aus -O- und - CH₂-, zum Verstärken des bzw. eines oder mehrerer angenehmer Geruchseindrücke eines oder mehrerer angenehm riechender Stoffe, wobei der/die Stoff(e) keine Verbindung der Formel (I) ist bzw. sind,
   wobei (i) die Verbindung der Formel (I) bzw. (ii) eine, mehrere oder sämtliche Verbindungen der Formel (I) ausgewählt ist bzw. sind aus der Gruppe bestehend aus Cyclohexadec-8-en-1-on (Aurelione, CAS No. 88642-039, 3100-36-5; Globanone, CAS No. 3100-36-5) und Cyclohexadecanon (Isomuscone, CAS No. 2550-52-9),
   und wobei der bzw. einer, mehrere oder sämtliche der angenehm riechenden, nicht der Formel (I) entsprechende(n) Stoff(e) ausgewählt ist bzw. sind aus der Gruppe bestehend aus Methyldihydrojasmonat, Benzylsalicylat, cis-3-Hexenylsalicylat, Isoamylsalicylat, Hexylsalicylat, 2,3,8,8-Tetramethyl-1,2,3,4,5,6,7,8-octahydro-2-naphthalenylmethylketon, Linalylacetat, Ethyllinalylacetat, Cedrylmethylether, Cedrylmethylketon, Cedrylacetat, (4aR,5R,7aS,9R)-Octahydro-2,2,5,8,8,9a-hexamethyl-4H-4a,9-methano azuleno(5,6-d)-1,3-dioxol), 1',1',5',5'-Tetra methyl-hexahydro-spiro[1,3-dioxolan-2.8'(5'H)-2H-2.4a]methanonaphthalin, Cyclododecylmethylether, (Ethoxymethoxy)cyclododecan, Decahydro-beta-naphthylacetat, 4,7-Methano-3a,4,5,6,7,7a-hexahydro-5(-6)-indenylacetat, Allyl-3-cyclohexylpropionat, Allylcyclohexyloxyacetat, Benzylbenzoat, Benzylcinnamat, 15-Hydroxy-Pentadecanonsäurelacton, 3-Methyl-cyclopentadecenon, 1,3,4,6,7,8-hexahydro-4,6,6,7,8,8-hexamethyl Cyclopenta[g]-2-benzopyran, 2-[1-(3,3-dimethylcyclohexyl)ethoxy]-2-methyl-,1-propanoat, 1,4-Dioxacycloheptadecan-5,17-dion, 3-Methy-cyclopentadecanon, 3a,6,6,9a-Tetramethyl dodecahydronaphtho[2,1-b]furan, alpha-Irone, beta-Irone, alpha-n-Methylionon, beta-n-Methylionon, alpha-Isomethylionon, beta-Isomethylionon und Allylionon, 2-Methyl-3-(4-tert-butylphenyl)propanal, 4-(4-Hydroxy-4-methylpentyl)-3-cyclohexencarboxaldehyd, 2-Methyl-4-(2,6,6-trimethyl-1-cyclohexen-1-yl)-2-butenal, 1-Methyl-4-(4-methyl-3-penten-1-yl)-3-cyclohexencarbox aldehyd, 3-(3-Isopropyl-phenyl)-butyraldehyd, (E)-2,6,10-Trimethyl-undeca-5,9-dienal, Benzo[1,3]dioxole-5-carbaldehyd, 2,2-Dimethyl-3-phenyl-propan-1-ol, 2,2-Dimethyl-3-m-tolyl-propan-1-ol, 1-(4-Isopropyl-cyclohexyl)-ethanol, (4-Isopropyl-cyclohexyl)-methanol, 2-Phenyl-ethanol, 2-Isobutyl-4-methyl-tetrahydro-pyran-4-ol, 3,7-Dimethyl-octa-1,6-dien-3-ol, (Z)-3,7-Dimethyl-octa-2,6-dien-1-ol, (E)-3,7-Dimethyl-octa-2,6-dien-1-ol, 3,7-Dimethyl-oct-6-en-1-ol, 2,6-Dimethyl-oct-7-en-2-ol, 3,7-Dimethyl-octan-1-ol, 2-Methyl-6-methylenoct-7-en-2-ol und (E/Z)-3,7-Dimethyl-nona-1,6-dien-3-ol..

Vorteilhafterweise eignen sich die Verbindungen der Formel (I) zudem zum Maskieren oder Vermindern des bzw. eines oder mehrerer unangenehmer Geruchseindrücke eines oder mehrerer unangenehm riechender (nicht der Formel (I) entsprechender) Stoffe.

Verbindungen der allgemeinen Formel (I) sind dem Fachmann grundsätzlich bekannt. Eine Beschreibung der geruchlichen Eigenschaften der Verbindungen der Formel (I) findet sich unter anderem in dem Fachbuch "S. Arctander, Perfume and Flavor Materials, Vol. I und II, Montclair, N. J., 1969, Selbstverlag" oder H. Surburg, J. Panten, Common Fragrance and Flavor Materials, 5th. Ed., Wiley-VCH, Weinheim 2006. Die hierin beschriebenen, der vorliegenden Erfindung zugrundeliegenden Effekte solcher Verbindungen bzw. Mischungen, insbesondere bei Kombination mit bestimmten weiteren Riechstoffen (wie weiter unten beschrieben) ist bisher nicht bekannt.

Überraschenderweise bewirken die Verbindungen der allgemeinen Formel (I), dass bestimmte gewünschte geruchliche Aspekte anderer Stoffe betont bzw. hervorgehoben/verstärkt und/oder bestimmte ungewünschte geruchliche Aspekte anderer Stoffe maskiert oder vermindert werden, vorzugsweise fettige, metallische und/oder (chemisch) technische Noten anderer (Riech-)Stoffe, insbesondere der hierin beschriebenen Riechstoffe.

Die erfindungsgemäß zu verwendenden Verbindungen der Formel (I) sind weiterhin vorteilhafterweise leicht zugänglich bzw. herstellbar, weisen bereits in geringer Konzentration eine hohe Wirksamkeit auf, insbesondere bei Konzentrationen, bei denen die Verbindungen der Formel (I) keinen oder zumindest lediglich einen kaum wahrnehmbarem Eigengeruch aufweisen, sind weitgehend oder vollständig farblos, besitzen eine hohe Stabilität in unterschiedlichen Mischungen bzw. Zubereitungen und weisen keine toxische und/oder allergene Wirkung gegenüber dem Menschen auf.

Verbindungen der Formel (I) haben zudem den Vorteil, dass sie bei ihrer Verwendung, insbesondere einer erfindungsgemäßen Verwendung, mit unterschiedlichen Riechstoffen und Parfümölen bzw. üblichen Bestandteilen eines Parfümöls kombiniert werden können, um Produkte mit einer beliebigen Duftrichtung zu parfümieren. Folglich lässt sich durch die vorliegende Erfindung den Verbrauchern gegenüber eine breite Auswahl an Dufttypen anbieten. Erfindungsgemäße Riechstoffmischungen und parfümierte Produkte enthaltend eine bzw. mehrere Verbindung(en) der Formel (I) werden weiter unten beschrieben.

Wie oben beschrieben, eignen sich Verbindungen der Formel (I) auch zum Maskieren oder Vermindern, v.a. zum Vermindern, des bzw. eines oder mehrerer unangenehmer Geruchseindrücke eines oder mehrerer unangenehm riechender, nicht der Formel (I) entsprechender Stoffe. Die hier genannten, "unangenehm riechenden Stoffe" können noch weitere, in der Regel nicht unangenehme sensorische Qualitäten, u.a. auch geruchliche Qualitäten, besitzen. Als "unangenehm riechende Stoffe" sind also im Rahmen des vorliegenden Textes generell Stoffe zu verstehen, die einen oder mehrere unangenehme Geruchseindrücke hervorrufen, sei es als primär-Geruch oder als neben-/untergeordnete Geruchsnote. Insbesondere können die hierin beschriebenen unangenehm riechenden Stoffe auch solche Stoffe sein, die in erster Linie als angenehm riechende Stoffe empfunden werden, jedoch (auch) eine metallische, fettige und/oder technische Note aufweisen, die es erfindungsgemäß bevorzugt zu reduzieren gilt. Entsprechendes gilt für die hierin beschriebenen "angenehm riechenden Stoffe" analog. D.h., als "angenehm riechende Stoffe" sind im Rahmen des vorliegenden Textes generell Stoffe zu verstehen, die einen oder mehrere angenehme Geruchseindrücke hervorrufen, sei es als primär-Geruch oder als neben-/untergeordnete Geruchsnote. Im Ergebnis kann im Rahmen der vorliegenden Erfindung beispielsweise sowohl eine unangenehme Geruchsnote eines bestimmten (unangenehm riechenden) Stoffes maskiert oder vermindert, als auch eine angenehme Geruchsnote desselben (auch angenehm riechenden) Stoffes verstärkt werden.

Erfindungsgemäß bevorzugt ist eine Verwendung wie oben beschrieben, wobei (i) die Verbindung der Formel (I) bzw. (ii) eine, mehrere oder sämtliche Verbindungen der Formel (I) ausgewählt ist bzw. sind aus der Gruppe bestehend aus Cyclohexadec-8-en-1-on (Aurelione, CAS No. 88642-03-9, 3100-36-5; Globanone, CAS No. 3100-36-5), Cyclohexadecanon (Isomuscone, CAS No. 2550-52-9), Oxacyclohexadecen-2-on (Globalide, CAS No. 34902-57-3, 111879-80-2), Cyclopentadecanolid (Macrolide, CAS No. 106-02-5) und (9Z)-17-Oxacycloheptadec-9-en-1-on (Ambrettolide, CAS No. 28645-51-4).

Die erfindungsgemäß zu verwendenden Verbindungen der Formel (I) und Mischungen davon eignen sich insbesondere zum Verstärken des bzw. eines oder mehrerer angenehmer Geruchseindrücke eines oder mehrerer angenehm riechender Stoffe, wobei der/die Stoff(e) keine Verbindung der Formel (I) ist bzw. sind, nämlich zum Verstärken bzw. Verbessern der natürlichen Frische und/oder der Ausstrahlung eines oder mehrerer angenehm riechender Stoffe und/oder zum Verstärken der bzw. einer blumigen Geruchsnote eines oder mehrerer angenehm riechender Stoffe, insbesondere einer Geruchsnote des Typs Jasmin.

Bei dem bzw. einem, mehreren oder sämtlichen Stoffen, deren geruchliche Eigenschaften durch Verwendung einer oder mehrerer Verbindungen der Formel (I) erfindungsgemäß verbessert werden, handelt es sich vorzugsweise um Riechstoffe mit einer blumigen Note, insbesondere des Typs Jasmin.

Grundsätzlich bevorzugt ist eine solche erfindungsgemäße Verwendung, wobei der bzw. einer, mehrere oder sämtliche der angenehm riechenden, nicht der Formel (I) entsprechende(n) Stoff(e) ausgewählt ist bzw. sind aus der Gruppe bestehend aus Riechstoffen mit einer Molmasse im Bereich von 150 g/mol bis 285 g/mol, insbesondere Alkoholen, Aldehyden, Ketonen, Ethern, Estern und Carboxylaten, insbesondere Alkoholen, Aldehyden, Ketonen, Ethern und Estern, mit einer Molmasse im Bereich von 150 g/mol bis 285 g/mol.

Besonders bevorzugt sind Stoffe, insbesondere Alkohole und Aldehyde, mit einer Molmasse von 210 g/mol oder weniger und Stoffe, insbesondere Ketone, Ether und Ester, mit einer Molmasse im Bereich von 190 g/mol bis 250 g/mol.

Im Rahmen der vorliegenden Erfindung werden der bzw. einer, mehrere oder sämtliche der angenehm riechenden, nicht der Formel (I) entsprechende(n) Stoff(e) ausgewählt bzw. sind aus der Gruppe bestehend aus Methyldihydrojasmonat, Benzylsalicylat, cis-3-Hexenylsalicylat, Isoamylsalicylat, Hexylsalicylat, 2,3,8,8-Tetramethyl-1,2,3,4,5,6,7,8-octahydro-2-naphthalenylmethylketon, Linalylace- tat, Ethyllinalylacetat, Cedrylmethylether, Cedrylmethylketon, Cedrylacetat, (4aR,5R,7aS,9R)-Octahydro-2.,2,5,8,8,9a- hexamethyl-4H-4a,9-methano azuleno(5,6-d)-1,3-dioxol), 1',1',5',5'-Tetra methyl-hexahydro-spiro[1.3-dioxolan-2.8'(5'H)-2H-2.4a]methanonaphthalin, Cyclododecylmethylether, (Ethoxymethoxy)cyclododecan, Decahydro-beta-naphthylacetat, 4,7-Methano-3a,4,5,6,7,7a-hexahydro-5(-6)-indenylacetat, Allyl-3-cyclohexylpropionat, Allylcyclohexyloxyacetat, Benzylbenzoat, Benzylcinnamat, 15-Hydroxy-Pentadecanonsäurelacton, 3-Methyl-cyclopentadecenon, 1,3,4,6,7,8-hexahydro-4,6,6,7,8,8-hexamethyl Cyclopenta[g]-2-benzopyran, 2-[1-(3,3-dimethylcyclohexyl)ethoxy]-2-methyl-,1-propanoat, 1,4-Dioxacycloheptadecan-5,17-dion, 3-Methy-cyclopentadecanon, 3a,6,6,9a-Tetramethyl dodecahydronaphtho[2,1-b]furan, alpha-Irone, beta-Irone, alpha-n-Methylionon, beta-n-Methylionon, alpha-Isomethylionon, beta-Isomethylionon und Allylionon, 2-Methyl-3-(4-tert-butylphenyl)propanal, 4-(4-Hydroxy-4-methylpentyl)-3-cyclohexencarboxaldehyd, 2-Methyl-4-(2,6,6-trimethyl-1-cyclohexen-1-yl)-2-butenal, 1-Methyl-4-(4-methyl-3-penten-1-yl)-3-cyclohexencarbox aldehyd, 3-(3-Isopropyl-phenyl)-butyraldehyd, (E)-2,6,10-Trimethyl-undeca-5,9-dienal, Benzo[1,3]dioxole-5-carbaldehyd, 2,2-Dimethyl-3-phenyl-propan-1-ol, 2,2-Dimethyl-3-m-tolyl-propan-1-ol, 1-(4-Isopropyl-cyclohexyl)-ethanol, (4-Isopropyl-cyclohexyl)-methanol, 2-Phenyl-ethanol, 2-Isobutyl-4-methyl-tetrahydro-pyran-4-ol, 3,7-Dimethyl-octa-1,6-dien-3-ol, (Z)-3,7-Dimethyl-octa-2,6-dien-1-ol, (E)-3,7-Dimethyl-octa-2,6-dien-1-ol, 3,7-Dimethyl-oct-6-en-1-ol, 2,6-Dimethyl-oct-7-en-2-ol, 3,7-Dimethyl-octan-1-ol, 2-Methyl-6-methylenoct-7-en-2-ol und (E/Z)-3,7-Dimethyl-nona-1,6-dien-3-ol.

Vorteilhafterweise sind die erfindungsgemäß zu verwendenden Verbindungen der Formel (I), insbesondere solche wie hierin als bevorzugt bezeichnet, besonders dazu geeignet, die natürliche Frische und/oder die Ausstrahlung dieser (Riech-) Stoffe zu verstärken bzw. zu verbessern und/oder eine blumige Geruchsnote, insbesondere eine Geruchsnote des Typs Jasmin, dieser (Riech-)Stoffe zu verstärken.

Besonders vorteilhaft ist eine erfindungsgemäße Verwendung, wobei das Verhältnis der Gesamtmasse an angenehm riechenden, nicht der Formel (I) entsprechenden Stoffen zur Gesamtmasse an Verbindung(en) der Formel (I) größer oder gleich 99 : 1 ist, bevorzugt größer oder gleich 99,9 : 0,1, besonders bevorzugt größer oder gleich 99,999 : 0,001.

Für die Zwecke der vorliegenden Erfindung besonders geeignet ist auch eine erfindungsgemäße Verwendung, wobei die zum Verstärken eingesetzte Menge an Verbindung(en) der Formel (I) nicht ausreicht, um einen Eigengeruch, insbesondere einen Moschusgeruch, zu vermitteln. Die erfindungsgemäß bevorzugten Verbindungen der Formel (I) haben nämlich den Vorteil, dass sie bei ihrer Verwendung, insbesondere einer erfindungsgemäßen Verwendung, bereits in solch geringen Konzentrationen für die Zwecke der vorliegenden Erfindung wirksam sind, bei denen kein oder zumindest kein wahrnehmbarer Eigengeruch der Verbindungen der Formel (I) vermittelt wird. Hierdurch können diese Verbindungen mit unterschiedlichen Riechstoffen sowie gegebenenfalls weiteren Bestandteilen eines Parfümöls kombiniert werden, um besonders vorteilhafte Riechstoffmischungen herzustellen oder um Produkte mit einer beliebigen Duftrichtung zu parfümieren.

Im Folgenden werden erfindungsgemäße Riechstoffmischungen beschrieben.

Dementsprechend betrifft ein weiterer Aspekt der vorliegenden Erfindung eine Riechstoffmischung, vorzugsweise ein Parfümöl, umfassend oder bestehend aus
(A)
   (i) einer einzelnen Verbindung der Formel (I) oder
   (ii) einer Mischung umfassend oder bestehend aus zwei oder mehr Verbindungen der Formel (I) 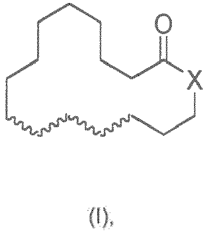 wobei für die Verbindung der Formel (I) bzw. jede Verbindung der Formel (I) gilt, dass
      keine, eine oder zwei der vier gewellten Linien eine Doppelbindung bedeutet bzw. bedeuten und die übrigen gewellten Linien jeweils eine Einfachbindung bedeuten und
      X ausgewählt ist aus -O-, -CH₂- und -O-CH₂-, vorzugsweise aus -O- und -CH₂-,
      und
(B) einem, zwei, drei, vier, fünf, sechs oder mehr (weiteren) Riechstoffen, wobei der bzw. die (weitere(n)) Riechstoff(e) keine Verbindung(en) der Formel (I) ist bzw. sind und
   einer, zwei, drei, vier, fünf, sechs oder mehr bzw. sämtliche der (weiteren) Riechstoffe eine Molmasse im Bereich von 150 g/mol bis 285 g/mol besitzt bzw. besitzen,
   dadurch gekennzeichnet, dass das Verhältnis der Gesamtmasse an nicht der Formel (I) entsprechenden Riechstoffen zur Gesamtmasse an Verbindung(en) der Formel (I) größer oder
   gleich 99,9 : 0,1, besonders bevorzugt größer oder gleich 99,999 : 0,001,
   wobei (i) die Verbindung der Formel (I) bzw. (ii) eine, mehrere oder sämtliche Verbindungen der Formel (I) ausgewählt ist bzw. sind aus der Gruppe bestehend aus Cyclohexadec-8-en-1-on (Aurelione, CAS No. 88642-03-9, 3100-36-5; Globanone, CAS No. 3100-36-5) und Cyclohexadecanon (Isomuscone, CAS No. 2550-52-9), und wobei der Riechstoff bzw. einer, mehrere oder sämtliche Riechstoffe gemäß Bestandteil (B) ausgewählt ist bzw. sind aus der Gruppe bestehend aus Methyldihydrojasmonat, Benzylsalicylat, cis-3-Hexenylsalicylat, Isoamylsalicylat, Hexylsalicylat, 2,3,8,8-Tetramethyl-1,2,3,4,5,6,7,8-octahydro-2-naphthalenylmethylketon, Linalylacetat, Ethyllinalylacetat, Cedrylmethylether, Cedrylmethylketon, Cedrylacetat, (4aR,5R,7aS,9R)-Octahydro-2,2,5,8,8,9a-hexamethyl-4H-4a,9-methano azuleno(5,6-d)-1,3-dioxol), 1',1',5',5'-Tetra methyl-hexahydro-spiro[1.3-dioxolan-2.8'(5'H)-2H-2.4a]methanonaphthalin, Cyclododecylmethylether, (Ethoxymethoxy)cyclododecan, Decahydro-beta-naphthylacetat, 4,7-Methano-3a,4,5,6,7,7a-hexahydro-5(-6)-indenylacetat, Allyl-3-cyclohexylpropionat, Allylcyclohexyloxyacetat, Benzylbenzoat, Benzylcinnamat, 15-Hydroxy-Pentadecanonsäurelacton, 3-Methyl-cyclopentadecenon, 1,3,4,6,7,8-hexahydro-4,6,6,7,8,8-hexamethyl Cyclopenta[g]-2-benzopyran, 2-[1-(3,3-dimethylcyclohexyl)ethoxy]-2-methyl-, 1-propanoat, 1,4-Dioxacycloheptadecan-5,17-dion, 3-Methy-cyclopentadecanon, 3a,6,6,9a-Tetramethyl dodecahydronaphtho[2,1b]furan, alpha-Irone, beta-Irone, alpha-n-Methylionon, beta-n-Methylionon, alpha-Isomethylionon, beta-Isomethylionon und Allylionon, 2-Methyl-3-(4-tert-butylphenyl)propanal, 4-(4-Hydroxy-4-methylpentyl)-3-cyclohexencarboxaldehyd, 2-Methyl-4-(2,6,6-trimethyl-1-cyclohexen-1-yl)-2-butenal, 1-Methyl-4-(4-methyl-3-penten-1-yl)-3-cyclohexencarbox aldehyd, 3-(3-Isopropyl-phenyl)-butyraldehyd, (E)-2,6,10-Trimethyl-undeca-5,9-dienal, Benzo[1,3]dioxole-5-carbaldehyd, 2,2-Dimethyl-3-phenyl-propan-1-ol, 2,2-Dimethyl-3-m-tolyl-propan-1-ol, 1-(4-isopropyl-cyclohexyl)-ethanol, (4-Isopropyl-cyclohexyl)-methanol, 2-Phenyl-ethanol, 2-Isobutyl-4-methyl-tetrahydropyran-4-ol, 3,7-Dimethyl-octa-1,6-dien-3-ol, (Z)-3,7-Dimethyl-octa-2,6-dien-1-ol, (E)-3,7-Dimethyl-octa-2,6-dien-1-ol, 3,7-Dimethyl-oct-6-en-1-ol, 2,6-Dimethyl-oct-7-en-2-ol, 3,7-Dimethyl-octan-1-ol, 2-Methyl-6-methylenoct-7-en-2-ol und (E/Z)-3,7-Dimethyl-nona-1,6-dien-3-ol..
Für die bevorzugt einzusetzenden bzw. enthaltenen Verbindungen der Formel (I) bzw. Mischungen davon gilt das oben Gesagte entsprechend. Für die Riechstoffe gemäß Bestandteil (B) gilt vorzugsweise das oben bezüglich der angenehm riechenden, nicht der Formel (I) entsprechenden Stoffe Gesagte entsprechend.
Der Fachmann wird in einer erfindungsgemäßen Riechstoffmischung (beispielsweise einem Parfümöl) den Anteil an Komponente (A), d. h. den Anteil an der Verbindungen der Formel (I), in Anbetracht der vorliegenden Textes so wählen, dass der von ihm gewünschte Effekt des Betonens/Verstärkens (Hervorhebens) einer Geruchsnote erreicht wird, wobei er bevorzugt Sorge tragen wird, keine zu große Menge der Komponente (A) einzusetzen, welche den sensorischen Gesamteindruck einer Riechstoffmischung dominieren könnte und andererseits nicht lediglich eine so geringe Menge der Komponente (A) vorzusehen, dass eine Betonung geruchlicher Aspekte von Riechstoffen der Komponente (B) nicht bzw. kaum mehr wahrgenommen werden kann.

Erfindungsgemäß besonders bevorzugte Mengenverhältnisse ergeben sich auch aus den Ausführungen weiter unten sowie insbesondere den beigefügten Beispielen.

Erfindungsgemäße Riechstoffmischungen sind üblicherweise bei 25°C und 1013hPa flüssig und in der Regel homogene Lösungen.

Riechstoffmischungen, insbesondere Parfümöle, umfassen häufig synthetische oder natürliche (vorzugsweise) geschmacks- und geruchsneutrale Trägeröle, welche die Duftbeziehungsweise Riechstoffe (als künstliche oder natürliche Stoffe) in hochkonzentrierter Form (sowie gegebenenfalls parfümistische Lösemittel und/oder Hilfsstoffe) enthalten.

Parfümöle dienen häufig Duftanwendungen. Mit Parfümölen werden beispielsweise Parfüme hergestellt, indem man sie in (z.B. alkoholische) Lösungen gibt, die beim Verdampfen die Duft- beziehungsweise Riechstoffe "mitreißen" und so dem Riechorgan des Anwenders, d.h. des Menschen, den Sinneseindruck eines bestimmten Geruchs vermitteln. Solche Mischungen können beispielsweise ein Parfüm, Eau de Parfüm oder Eau de Toilette sein. Ferner dienen Parfümöle der Erzeugung eines bestimmten Duftes in Wohnräumen, wie beispielsweise bei der Anwendung in Duftlampen, Zerstäubern oder Diffuser. Darüber hinaus können Parfümöle aber auch in unzähligen weiteren Artikeln bzw. Zubereitungen eingesetzt werden, beispielsweise von Schuhcremes bis Haarshampoos, Damenbinden bis WC-Reinigern, Gesichtscremes bis Waschpulver und Katzensteinen.

Eine erfindungsgemäße Riechstoffmischung kann neben den Bestandteilen (A) und (B) (wie oben beschrieben) ein oder mehrere weitere (übliche) Bestandteile bzw. Substanzen enthalten, auch ein oder mehrere weitere, nicht den vorstehenden Kriterien von Bestandteil (B) entsprechende Riechstoffe.

Beispiele für Riechstoffe, die grundsätzlich vorteilhaft als Bestandteil einer erfindungsgemäßen Riechstoffmischung, insbesondere eines erfindungsgemäßen Parfümöls, verwendet werden können, finden sich z.B. in S. Arctander, Perfume and Flavor Chemicals, Vol. I und II, Montclair, N. J., 1969, Selbstverlag oder H. Surburg, J. Panten, Common Fragrance and Flavor Materials, 5th Ed., Wiley-VCH, Weinheim 2006.

Bevorzugte Etherische Öle, Concretes, Absolues, Resine, Resinoide, Balsame und/oder Tinkturen, die Bestandteil einer erfindungsgemäßen Riechstoffmischung, insbesondere eines erfindungsgemäßen Parfümöls, sein können, sind bevorzugt auszuwählen aus der Gruppe bestehend aus:

Ambratinktur; Amyrisöl; Angelicasamenöl; Angelicawurzelöl; Anisöl; Baldrianöl; Basilikumöl; Baummoos-Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepreßt; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl (engl.: pine oil); Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Teebaum-Öl; Terpentinöl (engl.: turpentine oil); Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl.

Bevorzugte Einzelriechstoffe, die vorzugsweise als Bestandteil einer erfindungsgemäßen Riechstoffmischung, insbesondere eines erfindungsgemäßen Parfümöls, verwendet werden, sind ausgewählt aus der Gruppe der Kohlenwasserstoffe, dabei bevorzugt 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
der aliphatischen Alkohole, dabei bevorzugt Hexanol; Octanol; 3-Octanol; 2,6-Dimethylheptanol; 2-Methyl-2-heptanol; 2-Methyl-2-octanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
der aliphatischen Aldehyde und deren Acetale, dabei bevorzugt Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal;2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyloxyacetaldehyd; 1-(1-Methoxy-propoxy)-(E/Z)-3-hexen;
der aliphatischen Ketone und deren Oxime, dabei bevorzugt 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; 6-Methyl-5-hepten-2-on;
der aliphatischen schwefelhaltigen Verbindungen, dabei bevorzugt 3-Methylthio-hexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile, dabei bevorzugt 2-Nonensäurenitril; 2-Undecensäurenitril; 2-Tridecensäurenitril; 3,12-Tridecadiensäurenitril; 3,7-Dimethyl-2,6-octadien-säurenitril; 3,7-Dimethyl-6-octensäurenitril;
der Ester von aliphatischen Carbonsäuren, dabei bevorzugt (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;4-Methyl-2-pentyl-crotonat;
der acyclischen Terpenalkohole, dabei bevorzugt Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate;
der acyclischen Terpenaldehyde und -ketone, dabei bevorzugt Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
der cyclischen Terpenalkohole, dabei bevorzugt Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl-2-butenoate; Menthylformiat; Menthylpropionat; Menthylbutyrat; Menthylisobutyrat; Menthylisovalerianat; Menthylhexanoat; Menthylcrotonat; Menthyltiglinat;
der cyclischen Terpenaldehyde und -ketone, dabei bevorzugt Menthon; Isomenthon; 8-Mercaptomenthan-3-on; Carvon; Campher; Fenchon; alpha-Ionon; beta-Ionon; beta-n-Methylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on;2-Methyl-4-(2,6,6-trimethyl-1-cyclohexen-1-yl)-2-butenal; Nootkaton; Dihydronootkaton; 4,6,8-Megastigmatrien-3-on; alpha-Sinensal; beta-Sinensal; acetyliertes Cedernholzöl (Methylcedrylketon);
der cyclischen Alkohole, dabei bevorzugt 4-tert.-Butylcyclohexanol; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole, dabei bevorzugt alpha,3,3-Trimethylcyclohexylmethanol; 1-(4-Isopropylcyclohexyl)ethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether, dabei bevorzugt Cineol; Cedrylmethylether; Cyclododecylmethylether; 1,1-Dimethoxycyclododecan; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen und makrocyclischen Ketone, dabei bevorzugt 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 8-Cyclohexadecen-1-on; 9-Cycloheptadecen-1-on; Cyclopentadecanon; Cyclohexadecanon;
der cycloaliphatischen Aldehyde, dabei bevorzugt 2,4-Dimethyl-3-cyclohexencarbaldehyd; 2-Methyl-4-(2,2,6-trimethylcyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone, dabei bevorzugt 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole, dabei bevorzugt 2-tert-Butylcyclohexylacetat; 4-tert-Butylcyclohexylacetat; 2-tert-Pentylcyclohexylacetat; 4-tert-Pentylcyclohexylacetat; 3,3,5-Trimethylcyclohexylacetat; Decahydro-2-naphthylacetat; 2-Cyclopentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;
der Ester cycloaliphatischer Alkohole, vorzugsweise 1-Cyclohexylethylcrotonat;
der Ester cycloaliphatischer Carbonsäuren, dabei bevorzugt Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der araliphatischen Alkohole, dabei bevorzugt Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentan-1-ol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren, dabei bevorzugt Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
der araliphatischen Ether, dabei bevorzugt 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan; 4,4a,5,9b-Tetrahydroindeno[1,2-d]-m-dioxin; 4,4a,5,9b-Tetrahydro-2,4-dimethylindeno[1,2-d]-m-dioxin;
der aromatischen und araliphatischen Aldehyde, dabei bevorzugt Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 2-Methyl-3-(4-isobutylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone, dabei bevorzugt Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon;2-Benzofuranylethanon;(3-Methyl-2-benzofuranyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester, dabei bevorzugt Benzoesäure; Phenylessigsäure; Methylbenzoat; Ethylbenzoat; Hexylbenzoat; Methylphenylacetat; Ethylphenylacetat; Geranylphenylacetat; Phenylethylphenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen, dabei bevorzugt 2,4,6-Trinitro-1,3-dimethyl-5-tert.-butylbenzol; 3,5-Dinitro-2,6-dimethyl-4-tert.-butylacetophenon; Zimtsäurenitril; 3-Methyl-5-phenyl-2-pentensäurenitril; 3-Methyl-5-phenylpentansäurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin;2-(3-Phenylpropyl)pyridin; Indol; Skatol; 2-Methoxy-3-isopropyl-pyrazin; 2-Isobutyl-3-methoxypyrazin;
der Phenole, Phenylether und Phenylester, dabei bevorzugt Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen, dabei bevorzugt 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone, dabei bevorzugt 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid;4-Methyl-1,4-decanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,13-tridecandioat; Cumarin; 2,3-Dihydrocumarin; Octahydrocumarin.

Erfindungsgemäß ist eine Riechstoffmischung wie oben beschrieben, wobei (i) die Verbindung der Formel (I) bzw. (ii) eine, mehrere oder sämtliche Verbindungen der Formel (I) ausgewählt ist bzw. sind aus der Gruppe bestehend aus Cyclohexadec-8-en-1-on (Aurelione, CAS No. 88642-03-9, 3100-36-5; Globanone, CAS No. 3100-36-5) und Cyclohexadecanon (Isomuscone, CAS No. 2550-52-9).

Besonders bevorzugt ist eine erfindungsgemäße Riechstoffmischung, wobei die Menge an Verbindung(en) der Formel (I) in der Riechstoffmischung ausreicht, um den bzw. einen oder mehrere angenehme Geruchseindrücke des bzw. eines, mehrerer oder sämtlicher Riechstoffe gemäß Bestandteil (B) zu verstärken, insbesondere um die natürliche Frische und/oder Ausstrahlung des bzw. eines, mehrerer oder sämtlicher Riechstoffe gemäß Bestandteil (B) zu verstärken bzw. zu verbessern und/oder um die bzw. eine blumige Geruchsnote des bzw. eines, mehrerer oder sämtlicher Riechstoffe gemäß Bestandteil (B), vorzugsweise eine Geruchsnote des Typs Jasmin, zu verstärken.

Für die Zwecke der vorliegenden Erfindung ist eine solche erfindungsgemäße Riechstoffmischung beschrieben, wobei der Riechstoff bzw. einer, mehrere oder sämtliche Riechstoffe gemäß Bestandteil (B) ausgewählt ist bzw. sind aus der Gruppe bestehend aus Methyldihydrojasmonat, Benzylsalicylat, cis-3-Hexenylsalicylat, Isoamylsalicylat, Hexylsalicylat, 2,3,8,8-Tetramethyl-1,2,3,4,5,6,7,8-octahydro-2-naphthalenylmethylketon, Linalylacetat, Ethyllinalylacetat, Cedrylmethylether, Cedrylmethylketon, Cedrylacetat, (4aR,5R,7aS,9R)-Octahydro-2,2,5,8,8,9a-hexamethyl-4H-4a,9-methano azuleno(5,6-d)-1,3-dioxol), 1',1',5',5'-Tetra methyl-hexahydro-spiro[1.3-dioxolan-2.8'(5'H)-2H-2.4a]methanonaphthalin, Cyclododecylmethylether, (Ethoxymethoxy)cyclododecan, Decahydro-beta-naphthylacetat, 4,7-Methano-3a,4,5,6,7,7a-hexahydro-5(-6)-indenylacetat, Allyl-3-cyclohexylpropionat, Allylcyclohexyloxyacetat, Benzylbenzoat, Benzylcinnamat, 15-Hydroxy-Pentadecanonsäurelacton, 3-Methyl-cyclopentadecenon, 1,3,4,6,7,8-hexahydro-4,6,6,7,8,8-hexamethyl Cyclopenta[g]-2-benzopyran, 2-[1-(3,3-dimethylcyclohexyl)ethoxy]-2-methyl-,1-propanoat, 1,4-Dioxacycloheptadecan-5,17-dion, 3-Methy-cyclopentadecanon, 3a,6,6,9a-Tetramethyl dodecahydronaphtho[2,1-b]furan, alpha-Irone, beta-Irone, alpha-n-Methylionon, beta-n-Methylionon, alpha-Isomethylionon, beta-Isomethylionon und Allylionon, 2-Methyl-3-(4-tert-butylphenyl)propanal, 4-(4-Hydroxy-4-methylpentyl)-3-cyclohexencarboxaldehyd, 2-Methyl-4-(2,6,6-trimethyl-1-cyclohexen-1-yl)-2-butenal, 1-Methyl-4-(4-methyl-3-penten-1-yl)-3-cyclohexencarbox aldehyd, 3-(3-Isopropyl-phenyl)-butyraldehyd, (E)-2,6,10-Trimethyl-undeca-5,9-dienal, Benzo[1,3]dioxole-5-carbaldehyd, 2,2-Dimethyl-3-phenyl-propan-1-ol, 2,2-Dimethyl-3-m-tolyl-propan-1-ol, 1-(4-Isopropyl-cyclohexyl)-ethanol, (4-Isopropyl-cyclohexyl)-methanol, 2-Phenyl-ethanol, 2-Isobutyl-4-methyl-tetrahydro-pyran-4-ol, 3,7-Dimethyl-octa-1,6-dien-3-ol, (Z)-3,7-Dimethyl-octa-2,6-dien-1-ol, (E)-3,7-Dimethyl-octa-2,6-dien-1-ol, 3,7-Dimethyl-oct-6-en-1-ol, 2,6-Dimethyl-oct-7-en-2-ol, 3,7-Dimethyl-octan-1-ol, 2-Methyl-6-methylenoct-7-en-2-ol und (E/Z)-3,7-Dimethyl-nona-1,6-dien-3-ol.

Der Anteil an Verbindung(en) der Formel (I) beträgt in einer erfindungsgemäßen Riechstoffmischung, bezogen auf das Gesamtgewicht der Riechstoffmischung, 0,1 Gew.-% oder weniger, besonders bevorzugt 0,001 Gew.-% oder weniger.
Ein weiterer Aspekt der vorliegenden Erfindung betrifft ein parfümiertes Produkt, enthaltend eine erfindungsgemäße Riechstoffmischung (wie hierin beschrieben) bzw. ein oder mehrere Verbindungen der Formel (I), vorzugsweise ein erfindungsgemäßes Parfümöl (wie oben beschrieben), in einer sensorisch wirksamen Menge.
Dabei liegt der Anteil der Riechstoffmischung bezogen auf das Gesamtgewicht des Produkts im Bereich von 0,01 bis 10 Gew.-%, bevorzugt im Bereich von 0,1 bis 5 Gew.-%, besonders bevorzugt im Bereich von 0,25 bis 3 Gew.-%.
Für bevorzugt einzusetzende bzw. enthaltene Verbindungen der Formel (I) sowie gegebenenfalls die weiteren Bestandteile einer enthaltenen Riechstoffmischung gilt das oben Gesagte entsprechend.
Klarstellend ist zu erwähnen, dass erfindungsgemäße (parfümierte) Produkte im Rahmen des vorliegenden Textes als absichtlich herbeigeführte bzw. hergestellte Produkte zu verstehen sind, jedoch nicht als natürlich vorkommende Stoffmischungen wie sie zum Beispiel durch Extraktion von pflanzlichen Ausgangsmaterialien gewonnen werden können. Bevorzugte Produkte sind beispielsweise Parfüm-Extraits, Eau de Parfums, Eau de Toilettes, Rasierwässer, Eau de Colognes, Pre-shave-Produkte, Splash-Colognes und parfümierte Erfrischungstücher sowie parfümierte oder zu parfümierende saure, alkalische und neutrale Reinigungsmitteln, wie z.B. Fußbodenreiniger, Fensterglasreiniger, Geschirrspülmittel, Bad- und Sanitärreiniger, Scheuermilch, feste und flüssige WC-Reiniger, WC-Sticks, WC-Steine (flüssig oder fest), pulver- und schaumförmige Teppichreiniger, flüssige Waschmittel, pulverförmige Waschmittel, Wäschevorbehand- lungsmittel wie Bleichmittel, Einweichmittel und Fleckenentferner, Wäscheweichspüler, Waschseife, Waschtabletten, Desinfektionsmittel, Oberflächendesinfektionsmittel sowie Luftverbesserer in flüssiger, gelartiger oder auf einem festen Träger aufgebrachter Form, besonders zur Desodorierung von Abluft aus Klimaanlagen und industriellen Prozessen,
sowie Luftverbesserer in Form von Aerosol- oder Pumpsprays, Wachse und Polituren wie Möbelpolituren, Fußbodenwachse, Schuhcremes, stärkende, imprägnierende oder desodorierende Textilbehandlungsmittel, Windeln, Monatsbinden, Slip-Einlagen, Pflaster, sowie Körperpflegemittel wie z.B. feste und flüssige Seifen, Duschgele, Shampoos, Rasierseife, Rasierschäume, Badeöle, Feuchtreinigungstücher, kosmetische Emulsionen vom Öl-in-Wasser-, vom Wasser-in-Öl- und vom Wasser-in-Öl-in-Wasser-Typ wie z.B. Hautcremes und -lotionen, Gesichtscremes und -lotionen, Sonnenschutzcremes und - lotionen, After-sun-cremes und -lotionen, Handcremes und -lotionen, Fußcremes und - lotionen, Enthaarungscremes und -lotionen, After-shave-Cremes und -lotionen, Bräunungscremes und -lotionen,
Haarpflegepro- dukte wie z.B. Haarsprays, Haargele, festigende Haarlotionen, Haarspülungen, permanente und semipermanente Haar- färbemitteln, Haarverformungsmittel wie Kaltwellen- und Haarglättungsmittel, Haarwässer, Haarcremes und -lotionen, Deodorantien und Antiperspirantien wie z.B. Achsel-sprays, Roll-ons, Deosticks, Deocremes, Produkte der dekorativen
Kosmetik wie z.B. Lidschatten, Makeups, Lippenstifte, Mascara sowie Kerzen, Lampenöle, Räucherstäbchen, Tierstreu, Katzenstreu, Insektizide, Repellentien, flüssige und gasförmige Treibstoffe, Heizöle und Heizgase.

Besonders bevorzugt ist ein erfindungsgemäßes Produkt ausgewählt aus der Gruppe bestehend aus Parfüm-Extraits, Eau de Parfums, Eau de Toilettes, Rasierwässer, Eau de Colognes, Pre-shave-Produkte, Splash-Colognes, parfümierte Erfrischungstücher, saure, alkalische und neutrale Reinigungsmittel, Textilerfrischer, Bügelhilfen, flüssige Waschmittel, pulverförmige Waschmittel, Wäschevorbehandlungsmittel, Wäscheweichspüler, Waschseifen, Waschtabletten, Desinfektionsmittel, Oberflächendesinfektionsmittel, Luftverbesserer, Aerosolsprays, Wachse und Polituren, Körperpflegemittel, Handcremes und -lotionen, Fußcremes und -lotionen, Enthaarungscremes und -lotionen, After-shave-Cremes und -lotionen, Bräunungscremes und -lotionen, Haarpflegeprodukten, Deodorantien und Antiperspirantien, Produkte der dekorativen Kosmetik, Kerzen, Lampenöle, Räucherstäbchen, Insektizide, Repellentien und Treibstoffe.

Einem erfindungsgemäßen Produkt kann auch ein durch eine Verstärkung eines angenehmen geruchlichen Aspekts (insbesondere wie hierin beschrieben) geruchlich zu verbesserndes Produkt zugrundeliegen.

Gemäß einer bevorzugten Ausgestaltung sind die erfindungsgemäß einzusetzenden Verbindungen der Formel (I) an einem Trägerstoff adsorbiert, der sowohl für eine feine Verteilung der Verbindungen im Produkt als auch für eine kontrollierte Freisetzung bei der Anwendung sorgt. Derartige Träger können poröse anorganische Materialien wie Kieselgele, Zeolithe, Gipse, Tone, Tongranulate, Gasbeton usw. oder organische Materialien wie Hölzer und Cellulose-basierende Stoffe sein.

Die erfindungsgemäß einzusetzenden Verbindungen der Formel (I) beziehungsweise entsprechende Mischungen davon oder Riechstoffmischungen (wie hierin beschrieben) können auch mikroverkapselt, sprühgetrocknet, als Einschluss-Komplexe oder als Extrusions-Produkte vorliegen und in dieser Form einem Produkt hinzugefügt werden.

Gegebenenfalls können die Eigenschaften solcher derart modifizierten erfindungsgemäß zu verwendenden Verbindungen der Formel (I) durch sogenanntes "Coaten" mit geeigneten Materialien im Hinblick auf eine gezieltere Freisetzung weiter optimiert werden, wozu vorzugsweise wachsartige Kunststoffe wie z.B. Polyvinylalkohol verwendet werden.

Eine Mikroverkapselung der erfindungsgemäß einzusetzenden Verbindungen der Formel (I) kann beispielsweise durch das sogenannte Koazervationsverfahren mit Hilfe von Kapselmaterialien, z.B. aus polyurethan-artigen Stoffen oder Weichgelatine, erfolgen. Sprühgetrocknete Verbindungen der Formel (I) können beispielsweise durch Sprühtrocknung einer erfindungsgemäß einzusetzende Substanz, d.h. einer einen Alkohol der Verbindung der Formel (I) oder eine entsprechende Mischung enthaltenden Emulsion bzw. Dispersion hergestellt werden, wobei als Trägerstoff modifizierte Stärken, Proteine, Dextrin und/oder pflanzliche Gummen verwendet werden können. Einschluss-Komplexe können z.B. durch Eintragen von Dispersionen, welche erfindungsgemäß einzusetzende Verbindungen der Formel (I) oder entsprechende Mischungen davon sind beziehungsweise solche umfassen, und Cyclodextrinen oder Harnstoffderivaten in ein geeignetes Lösungsmittel, z.B. Wasser, hergestellt werden. Extrusions-Produkte können durch Verschmelzen der erfindungsgemäß einzusetzenden Verbindung(en) der Formel (I) beziehungsweise entsprechender Mischungen mit einem geeigneten wachsartigen Stoff und durch Extrusion mit nachfolgender Erstarrung, ggf. in einem geeigneten Lösungsmittel, z.B. Isopropanol, erfolgen.

Die erfindungsgemäß einzusetzenden Verbindungen der Formel (I) beziehungsweise entsprechende Mischungen können in vielen Zubereitungen bzw. Produkten eingesetzt werden, wobei sie vorzugsweise mit einem oder mehreren der folgenden Hilfs- oder Wirkstoffe kombiniert werden:

Konservierungsmittel, Abrasiva, Antiakne-Mittel, Mittel gegen Hautalterung, anitbakterielle Mittel, Anticellulitis-Mittel, Antischuppen-Mittel, entzündungshemmende Mittel, irritationsverhindernde Mittel, irritationshemmende Mittel, antimikrobielle Mittel, Antioxidantien, Adstringentien, schweisshemmende Mittel, antiseptische Mittel, Antistatika, Binder, Puffer, Trägermaterialien, Chelatbildnder, Zellstimulantien, reinigende Mittel, pflegende Mittel, Enthaarungsmittel, oberflächenaktive Substanzen, deodorierende Mittel, Antiperspirantien, Weichmacher, Emulgatoren, Enzyme, ätherische Öle, Fasern, Fixateure, Schaumbildner, Schaumstabilisatoren, Substanzen zum Verhindern des Schäumens, Schaumbooster, Fungizide, gelierende Mittel, gelbildende Mittel, Haarpflegemittel, Haarverformungsmittel, Haarglättungsmittel, feuchtigkeitsspendende Mittel, anfeuchtende Substanzen, feuchthaltende Substanzen, bleichende Mittel, (Textil-)stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, Pulver, Proteine, rückfettende Mittel, abschleifende Mittel, Silicone, hautberuhigende Mittel, hautreinigende Mittel, hautpflegende Mittel, hautheilende Mittel, Hautaufhellungsmittel, hautschützende Mittel, hauterweichende Mittel, kühlende Mittel, hautkühlende Mittel, wärmende Mittel, hautwärmende Mittel, Stabilisatoren, UVabsorbierende Mittel, UV-Filter, Waschmittel, Weichspüler, suspendierende Mittel, Hautbräunungsmittel, Verdickungsmittel, Vitamine, Öle, Wachse, Fette, Phospholipide, gesättigte Fettsäuren, ein- oder mehrfach ungesättigte Fettsäuren, α-Hydroxysäuren, Polyhydroxyfettsäuren, Verflüssiger, Farbstoffe, farbschützende Mittel, Pigmente, Antikorrosiva, Aromen, Geschmackstoffe, Riechstoffe, Polyole, Tenside, Elektrolyte, organische Lösungsmittel oder Silikonderivate.

Gemäß einer Ausgestaltung der vorliegenden Erfindung enthält ein bevorzugtes erfindungsgemäßes Produkt, insbesondere ein Deodorant oder dergleichen, (je nach gewünschter Wirkweise) zudem einen oder mehrere der folgenden Wirkstoffe:
(1) Antimikrobiell wirksame Substanzen, die die Entwicklung der für Schweißgeruch verantwortlichen Mikroorganismen hemmen; beispielsweise Triclosan^{®} (5-Chlor-2-(2,4-dichlorphenoxy)phenol), Triclocarban, Chlorhexidin, Chlorhexidinhydrochlorid, Chlorhexidindiacetat, Chlorhexidindigluconat, 2-Phenoxyethanol, Farnesol, Glycerinester und -ether wie Glycerinmonolaurat, Glycerinmonocaprinat, Hexoxyglycerin, Octoxyglycerin (= Ethylhexylglycerin, 3-(2-Ethylhexyloxy-1,2-Propandiol) oder Sensiva^{®} SC 50 (von Schülke & Mayr), aliphatische 1,2-Diole wie z.B. 1,2-Decandiol (EP 1 269 983), araliphatische Alkohole wie beispielsweise beschrieben in EP 799 174, vorzugsweise 4-Methyl-4-phenyl-2-pentanol (Vetikol; WO 03/024907) oder 2-Methyl-4-phenyl-2-butanol (1,1-Dimethyl-3-phenylpropanol, alpha,alpha-Dimethylphenethylcarbinol), I-Menthylmethylether wie beschrieben in WO 02/41861, 2-Benzylheptan-1-ol (Jasmol; 2-n-Pentyl-3-phenylpropan-1-ol), 2,2-Dimethyl-3-phenylpropanol (Muguetalkohol; vgl. US 4,091,090), antimikrobiell wirksame sekundäre Alkohol, wie beispielsweise beschrieben in WO 2005/004601, insbesondere 3-Methyl-6-phenyl-2-hexanol, 4-(2,4-Dimethylphenyl)-2-butanol, 6-(4-Isopropylphenyl)-3-methyl-2-hexanol, 4-(2,4,5-Trimethylphenyl)-2-butanol, 3,3-Dimethyl-4-phenyl-2-butanol, 3-Methyl-4-(2-methylphenyl)-2-butanol, 6-(3,4-Dimethylphenyl)-2-hexanol, aliphatische Carbonsäuren wie 2-Hexyloctansäure, 2-Hexyldecansäure, 2-Butyloctansäure oder 2-Butyldecansäure;
(2) enzyminhibierende Substanzen, die die Wirkung von Enzymen, die an der Bildung von Schweißgeruch beteiligt sind, unterbinden; beispielsweise Citronensäureester und metall-chelatisierende Substanzen wie EDTA (Ethylendiamintetraessigsäure), EGTA [Ethylenbis(oxyethylennitrilo)-tetraessigsäure] und DTPA (Diethylentriaminpentaessigsäure, Pentetic acid);
(3) geruchsabsorbierende Substanzen, die für den Schweißgeruch verantwortliche Stoffe absorbieren; beispielsweise Zinkrizinoleat, Cyclodextrine;
(4) Antiperspirantien, welche die Schweißsekretion hemmen und damit den für den Körpergeruch verantwortlichen Bakterien den Nährboden entziehen. Als Antiperspirantien verwendet man im allgemeinen vorzugsweise adstringierende Metallsalze, besonders anorganische und organische Metallsalze der Elemente Aluminium, Zink, Magnesium, Zinn und Zircon sowie deren Mischungen, wobei insbesonders Halogenide wie Aluminiumchlorid, basische Aluminiumhydroxychloride, Zirconyloxychloride und Zirconylhydroxychloride sowie deren Mischungen verwendet werden. Häufig werden diese Aluminium- und Zirconiumsalze und deren Mischungen auch in einer komplexierten Form verwendet, wobei als Komplexbildner vorzugsweise Propylenglykol, Polyethylenglykol oder Glycin verwendet werden.

Die vorliegende Erfindung betrifft weiterhin ein Verfahren zum Herstellen eines parfümierten Produkts, insbesondere eines erfindungsgemäßen parfümierten Produkts (wie hierin beschrieben), umfassend folgende Schritte:
i) Bereitstellen der Bestandteile (A) und (B) wie oben definiert, vorzugsweise Bereitstellen einer erfindungsgemäßen Riechstoffmischung wie oben beschrieben, insbesondere einer erfindungsgemäßen Riechstoffmischung wie hierin als bevorzugt bezeichnet,
ii) Bereitstellen eines oder mehrerer weiterer Bestandteile des herzustellenden parfümierten Produkts, und
iii) Inkontaktbringen oder Mischen der in Schritt ii) bereitgestellten weiteren Bestandteile mit einer sensorisch wirksamen Menge der in Schritt i) bereitgestellten Bestandteile (A) und (B) bzw. der in Schritt i) bereitgestellten Riechstoffmischung.

Im vorliegenden Zusammenhang ist auch ein Verfahren relevant, mit welchem beurteilt werden kann, ob es sich bei einer bestimmten Substanz (Stoff oder Mischung) um eine geeignete Substanz für eine bestimmte Aufgabe handelt, d.h. ob durch Verwendung dieser Substanz ein unangenehmer Geruch im Sinne der vorliegenden Erfindung maskiert bzw. vermindert und/oder ein angenehmer Geruchseindruck verstärkt werden kann.

Im Rahmen der vorliegenden Erfindung wird zum Testen der Eignung einer Testsubstanz (umfassend oder bestehend aus einer oder mehreren Verbindungen der Formel (I)) zum Maskieren oder Vermindern eines unangenehmen Geruchs bevorzugt ein Verfahren eingesetzt, das folgende Schritte umfasst:
a) Herstellen oder Bereitstellen einer (Malodor-)Standard-Mischung (unangenehm riechende Standard-Mischung) umfassend oder bestehend aus einer Substanz (Stoff oder Mischung) mit einem unangenehmen Geruch,
b) Herstellen oder Bereitstellen einer Testmischung ebenfalls umfassend die Substanz mit einem unangenehmen Geruch sowie zudem die zu untersuchende Testsubstanz, und
c) Vergleich der (Malodor-)Eindrücke der (Malodor-)Standard-Mischung und der Testmischung.

Ein solches Verfahren kann in analoger Weise zum Testen der Eignung einer Testsubstanz (umfassend oder bestehend aus einer oder mehreren Verbindungen der Formel (I)) zum Verstärken eines angenehmen Geruchseindrucks angewendet werden.

Vorzugsweise wird ein vorstehend beschriebenes Testverfahren bei einer Temperatur von 20 °C und einem Druck von 1013 mbar durchgeführt.

Bevorzugt wird das Testverfahren so durchgeführt, dass die Konzentrationen der unangenehm (oder angenehm) riechenden Substanz in der (Malodor-)Standard-Mischung und der Testmischung gleich groß sind. Dies erlaubt einen besonders guten Vergleich der beiden Mischungen.

Die (Malodor-)Eindrücke der (Malodor-)Standard-Mischung und der Testmischung sowie vorzugsweise auch die Parfüm- bzw. Duftintensität dieser Mischungen wird dabei vorzugsweise jeweils von 8 oder mehr Prüfern (Experten) durch Riechen im Vergleich zueinander bewertet. Zum Beispiel wird dabei einer Malodorstandard-Mischung die Intensität 6 zugeordnet.

Die Prüfer werden aufgrund ihrer Fähigkeit, die Stärken von Gerüchen reproduzierbar zu bewerten, ausgewählt und vor der Testreihe auf das Erkennen der (zu reduzierenden bzw. zu verstärkenden) unangenehmen bzw. angenehmen Gerüche trainiert.

Im Folgenden wird die Erfindung anhand ausgewählter Beispiele näher erläutert. Die Beispiele dienen zur Verdeutlichung der Erfindung, ohne den Schutzbereich der Patentansprüche einzuschränken. Prozentangaben beziehen sich auf das Gewicht, sofern nicht anders angegeben. Die mit * bezeichneten Ausführungsbeispiele sind erfindungsgemäß.

### Beispiel 1: Geruchsbeschreibung ausgewählter Riechstoffe nach Zugabe augewählter Verbindungen der Formel (I)

| **Riechstoff** | **Verbindung der Formel (I)** | **Massenverhältnis Riechstoff : Verbindung der Formel (I)** | **Geruchsbeschreibung im Vergleich zum Geruch des reinen Riechstoffs** |
|---|---|---|---|
| Mugetanol (M=170 g/mol) | a) Globanone, CAS No. 3100-36-5 | a) 100 : 0,5 | weicher, natürlicher, blumiger, weniger technisch |
| | b) Globalide, CAS No. 34902-57-3 | b) 100 : 0,1 | |
| Dihydromyrcenol (M=156 g/mol) | a) Globalide, CAS No. 34902-57-3 | a) 100 : 0,1 | weniger technisch, mehr natürlich-lavendelartig, blumiger |
| | b) Globanone, CAS No. 3100-36-5 | b) 100 : 0,2 | |
| Linalool (M=154 g/mol) | Isomuscone*, CAS No. 2550-52-9 | 100 : 0,1 | weniger technisch, frischer, natürlicher |
| Geraniol (M=154 g/mol) | Globalide, CAS No. 34902-57-3 | 100 : 0,1 | weniger fettig, weniger metallisch, runder, natürlicher, mehr Volumen |
| Citronellol (M=156 g/mol) | a) Globalide, CAS No. 34902-57-3 | a) 100 : 0,05 | weniger fettig, natürlicher, mehr rosig |
| | b) Globanone*, CAS No. 3100-36-5 | b) 100 : 0,1 | |
| Phenoxanol (M=178 g/mol) | a) Globalide, CAS No. 34902-57-3 | a) 100 : 0,1 | weniger technisch, mehr rosiger, natürlicher |
| | b) Globanone, CAS No. 3100-36-5 | b) 100 : 0,2 | |
| Lilial^{®} (M=204 g/mol) | Globalide, CAS No. 34902-57-3 | 100 : 0,1 | weniger technisch, frischer, natürlicher, stärker nach Maiglöckchen riechend |
| Aldehyd MNA (M=184 g/mol) | Globalide, CAS No. 34902-57-3 | 100 : 0,1 | weniger fettig, weniger metallisch, runder |
| Melonal^{®} (M=140 g/mol) | Ambrettolide, CAS No. 28645-51-4 | 100 : 0,1 | weniger technisch, weniger metallisch, runder, natürlicher |
| para tert.-Butyl-cyclohexanon (M=154 g/mol) | Globanone, CAS No. 3100-36-5 | 100 : 0,1 | weniger technisch, runder, harmonischer, mehr nach Patchouliöl riechend, frischer |
| Oryclon^{®} (M=198 g/mol) | Globalide, CAS No. 34902-57-3 | 100 : 0,1 | weniger technisch, runder, harmonischer, frischer |
| Mayol | Isomuscone* , CAS No. 2550-52-9 | 100 : 0,1 | weniger technisch, weniger animalisch, runder, harmonischer, frischer |
| Citronallal | Globalide, CAS No. 34902-57-3 | 100 : 0,1 | weniger technisch metallisch, mehr rosig, natürlicher |
| Majantol | Macrolide, CAS No. 106-02-5 | 100 : 0,1 | weniger technisch, runder, harmonischer, frischer |
| Aldehyd C14 | Globanone, CAS No. 3100-36-5 | 100 : 0,1 | weniger technisch, runder, harmonischer, blumiger |
| Ligustrai^{®} | a) Macrolide, CAS No. 106-02-5 | a) 100 : 0,05 | weniger technisch, runder, harmonischer, mehr Volumen |
| | b) Globanone, CAS No. 3100-36-5 | b) 100 : 0,1 | |
| Agrumex^{®} | a) Globalide, CAS No. 34902-57-3 | a) 100 : 0,05 | weniger technisch, runder, natürlicher, fruchtiger |
| | b) Globanone, CAS No. 3100-36-5 | b) 100 : 0,1 | |

Die in der Beschreibung sowie in obiger Tabelle beschriebenen geruchlichen Effekte der Verbindungen der Formel (I) gegenüber (anderen) Riechstoffen wurden jeweils auch bei den nachfolgend beschriebenen Anwendungsbeispielen beobachtet. Nachfolgende Beispiele sind nicht erfindungsgemäß.

### Beispiel 2: Anwendungsbeispiele

Für die unten beschriebenen Formulierungen kann je nach Belieben eines der folgenden Parfümöle eingesetzt werden:

**Parfümöl P1 :**

| | |
|---|---|
| Aldehyde C 12 Laurin | 3,5 |
| Decenol-9 | 0,5 |
| Vertocitral | 7,5 |
| Dihydro Myrcenol | 170,0 |
| Citronitril | 0,6 |
| Orangenoel Terpene | 30,0 |
| Eucalyptol | 10,0 |
| Beifussoel | 1,5 |
| Kampfer DL | 10,0 |
| Aldehyd C 14 sog. | 4,5 |
| Benzophenon | 10,0 |
| Benzyl Acetate | 25,0 |
| Hexylzimtaldehyd alpha | 10,00 |
| Agrumex HC | 350,0 |
| Oryclon HC | 100,0 |
| Herbaflorat | 45,0 |
| Herbylpropionat | 75,0 |
| | |
| Dipropylenglycol | 146,9 |
| | 1.000,00 |

Mit Zusatz von 0,5 Teilen von Globalide, CAS No. 34902-57-3, (zum Erhalt eines Par- fümöls) wird die Mischung runder, harmonischer, natürlicher, stärker und nicht mehr so technisch.

Mit Zusatz von 1 Teil von Globanone, CAS No. 3100-36-5, (zum Erhalt eines Parfümöls) wird die Mischung in der Top-Note stärker und ergibt eine zusätzliche blumige Note.

**Parfümöl P2:**

| | |
|---|---|
| Undecavertol | 2,5 |
| Dimethylbenzylcarbinylacetat | 35,0 |
| Agrumex HC | 200,0 |
| Hexenylacetat cis-3 | 7,5 |
| Vertocitral | 15,0 |
| Oxanthia 50% in TEC | 3,5 |
| Decalacton Gamma | 150,0 |
| Aldehyd C18 sog. | 35,0 |
| Aldehyd C14 sog. | 350,0 |
| Thiomenthanon-8,3 0,1% in DPG | 3,5 |
| Orangenoel Bras. | 75,0 |
| Hedion | 100,0 |
| Ethylmethylbutyrat-2 | 1,5 |
| Benzylacetat | 20,0 |
| Damascon alpha | 1,5 |
| | |
| | 1.000,00 |

Mit Zusatz von 0,2 Teilen von Macrolide, CAS No. 106-02-5, (zum Erhalt eines Parfümöls) wird die Mischung runder nicht mehr so technisch, frischer.

Mit Zusatz von 0,5 Teilen von Globalide, CAS No. 34902-57-3, (zum Erhalt eines Par- fümöls) wirkt die Mischung harmonischer mehr blumiger und stärker in Richtung Jasmin.

**Parfümöl P3:**

| | |
|---|---|
| Acetessigsäureethylester | 40,0 |
| Hexenol cis-3 | 2,0 |
| Terpinylacetat | 10,0 |
| Fir Balsam abs. 10% in BB | 5,0 |
| Jasmoprunat | 50,0 |
| Ethylisovalerianat | 6,8 |
| Isoamylisovalerianat | 1,4 |
| Ethylcaprylat | 6,0 |
| Ethylcaprinat | 4,0 |
| Aldehyd C14 sog. | 50,0 |
| Aldehyd C18 sog. | 3,4 |
| Aldehyd C16 sog. | 40,0 |
| Frambinon^{®} | 50,0 |
| Ethylmaltol | 20,0 |
| Linalool | 300,0 |
| Dimethylbenzylcarbinylbutyrat | 10,0 |
| Phennirat^{®} | 60,0 |
| Geranylbutyrat | 10,0 |
| Damascon alpha | 0,5 |
| Benzylacetat | 60,0 |
| Iraldein gamma | 50,0 |
| Anisylacetat | 10,0 |
| Vanillin | 40,0 |
| Benzaldehyd | 30,0 |
| Agrumex LC | 40,0 |
| Dipropylene glycol | 100,9 |
| | |
| | 1000,00 |

Mit Zusatz von 0,5 Teilen von Globalide, CAS No. 34902-57-3, (zum Erhalt eines Parfümöls) wird die Mischung runder, nicht mehr so technisch und mehr natürlicher.

Mit Zusatz von 1 Teil von Isomuscone, CAS No. 2550-52-9, (zum Erhalt eines Parfümöls) wirkt die Mischung mehr blumiger und harmonischer.

**Parfümöl P4:**

| | |
|---|---|
| Aldehyde C10 | 60,0 |
| Aldehyde C11 MOA | 5,0 |
| Florazon | 5,0 |
| Ozonil | 1,0 |
| Scentenal | 3,0 |
| Vertocitral | 3,0 |
| vertosine | 1,0 |
| Dynascone Melange 1:1 | |
| 10% ig in DPG | 2,0 |
| Mintonat | 25,0 |
| Dihydromyrcenol | 150,0 |
| Terpinylacetat | 50,0 |
| Litsea Cubebaoel | 5,0 |
| Agrunitril | 6,0 |
| Citrowanil^{®} B | 5,0 |
| Citronellal | 3,0 |
| Citrylal | 11,0 |
| Nerolione | 1,0 |
| Lavandinoel Grosso | 2,0 |
| Eucalyptusoel Globulus | 5,0 |
| Menthol rac. | 120,0 |
| Krauseminzoel | 2,0 |
| Herboxan | 8,0 |
| Thymol | 4,0 |
| Pineoel | 200,0 |
| Isobornylacetat | 110,0 |
| Aldehyd C14 sog. | 3,0 |
| Cyclamenaldehyd | 1,0 |
| Tetrahydrolinalool | 1,0 |
| Terpineol | 10,0 |
| Phenylethylacetat | 2,0 |
| Citronellol | 10,0 |
| Diphenyloxid | 10,0 |
| Benzylacetat | 10,0 |
| Methylbenzoat | 2,0 |
| Hexylsalicylat | 100,0 |
| lonon beta | 1,0 |
| Heliotropin | 3,0 |
| Agrumex HC | 25,0 |
| Orylclon | 310,0 |
| Herbaflorat | 38,0 |
| Koavone | 10,0 |
| Ambrocenide^{®} 10% in DPGI | 2,0 |
| BHT Jonol | 25,0 |
| Dipropylenglycol | 250,0 |
| | |
| | 1000,0 |

Mit Zusatz von 0,5 Teilen von Globalide, CAS No. 34902-57-3, (zum Erhalt eines Par- fümöls) wird die Mischung runder, blumiger nicht mehr so technisch.

Mit Zusatz von 1 Teil Ambrettolide, CAS No. 28645-51-4, (zum Erhalt eines Parfümöls) riecht die Mischung nicht mehr so technisch und erlangt mehr Volumen.

**Parfümöl P5:**

| | |
|---|---|
| Vertocitral | 1,0 |
| Galbanum Artessence | 3,0 |
| Alllyamylglycolat^{®} | 5,0 |
| Cyclogalbanat^{®} | 10,0 |
| Bergamot Identoil^{®} colorless | 100,0 |
| Dihydrmyrcenol | 200,0 |
| Terpinylacetat | 100,0 |
| Citral | 10,0 |
| Geranylnitril Replacer | 3,0 |
| Petigrainoel | 10,0 |
| methylnaphthylketon | 5,0 |
| Lavandinoel Grosso | 30,0 |
| Rosmarinoel | 10,0 |
| Pfefferminzoel Arv, DMO | 5,0 |
| Fir Balsam abs. 10% DPG | 1,0 |
| Kiefernnadel abs. | 0,6 |
| Hexylacetat | 1,0 |
| Decalacton delta 1 % DEP | 0,5 |
| Maltol 1% DPG | 10,0 |
| Calone 1951 0,1% DPG | 0,5 |
| Hydroxycitronellal | 25,0 |
| Geraniumoel | 5,0 |
| Citronellol | 10,0 |
| Damascon alpha | 2,0 |
| Hedion | 10,0 |
| Hexylzimtaldehydalpha | 44,0 |
| Boronal 10% DPG | 1,0 |
| Isoraldein 70 | 10,1 |
| Oryclon HC | 17,5 |
| Oryclon spezial | 32,5 |
| Cedernholzoel 10% DPG | 2,0 |
| Caryophyllenacetat | 17,6 |
| Vertofix 10% DPG | 2,0 |
| Cedrylacetat | 2,1 |
| Palisandal | 35,0 |
| Vetiveroel 10% DPG | 1,0 |
| Sandelwoodoil Melange 1 10% DPG | 2,0 |
| Isobornylcyclohexanol | 4,2 |
| Evernyl | 2,1 |
| Mousse C abs. 70A757 | 1,0 |
| Ambrinol S 10% DPG | 2,0 |
| Ambroxid 10% IPM | 2,0 |
| Ketamber 10% TEC | 2,0 |
| Hercolyn D-E | 34,2 |
| Dipropylenglycol | 228,1 |
| | 1000,0 |

Mit Zusatz von 0,5g von Globalide, CAS No. 34902-57-3, (zum Erhalt eines Parfümöls) wird die Mischung runder, nicht so technisch, frischer und mehr natürlicher.

Mit Zusatz von 1g von Globanone, CAS No. 3100-36-5, (zum Erhalt eines Parfümöls) erhält die Mischung mehr Volumen und wirkt natürlicher.

### Beispiel F1 - Waschpulver

| **Material** | **Chemischer Name** | **Funktion** | **Gew.-%** | **Gew.-%** |
|---|---|---|---|---|
| Natrium Metasilicat Pentahydrat | Sodium Metasilicate Pentahydrate | | Ad 100 | Ad 100 |
| Natriumhydrogencarbonat | Sodium hydrogen carbonate | Alkali | 15,0 | 15,0 |
| Natriumpercarbonat | Sodium carbonate peroxyhydrate | Bleichmittel | 15,0 | 15,0 |
| Peractive AC Blue | TAED / Na-Carboxy methylcellulose | Aktivator | 5,00 | 5,00 |
| Genapol OA-080 | Oxoalkohol C14-15, 8EO | Nichtionisches Tensid | 3,00 | 3,00 |
| Texapon K12 Pulver | Sodium Lauryl Sulphate C12 | Anionisches Tensid | 7,00 | 7,00 |
| Tinopal CBS-X | | Aufheller | 0,50 | 0,50 |
| Savinase 6.0 T, Type W | Protease | Enzym | 0,40 | 0,40 |
| Termamyl 120 T | Alpha-Amylase | Enzym | 0,30 | 0,30 |
| Natriumsulfat | Sodium Sulphate | Füllstoff | 5,50 | 5,50 |
| Parfumöl gemäß P1, P2, P3, P4 oder P5 | | Parfum (Fragrance) | 0,30 | 0,50 |

### Beispiel F2 - Allzweckreiniger

| **Material** | **Chemischer Name** | **Funktion** | **Gew.-%** | **Gew.-%** |
|---|---|---|---|---|
| Entionisiertes Wasser | Water | Lösungsmittel | Ad 100 | Ad 100 |
| Mergal K9N | 5-Chloro-2-methyl-3-(2H)-isothiazolone und 2-methyl-3-(2H)-isothiazolone | Konservierungsmittel | 0,1 | 0,1 |
| Trinatriumcitrat Dihydrat | Tri Sodium Citrate Dihydrate | Komplexbildner | 3,0 | 3,0 |
| Zetesol NL-2 | Fatty alcohol C12-14-sulfate, Sodium | Anionisches Tensid | 30,0 | 30,0 |
| Imbentin C/125/055 | Fatty alcohol C12-C15, 8EO | Nichtionisches Tensid | 5,0 | 5,0 |
| Ethanol | Ethanol | Lösungsmittel | 2,0 | 2,0 |
| Parfumöl gemäß P1, P2, P3, P4 oder P5 | | Parfum (Fragrance) | 0,3 | 0,5 |

### Beispiel F3 - Shampoo

| **Material** | **INCI-Name** | **Gew.-%** | **Gew.-%** |
|---|---|---|---|
| Entionisiertes Wasser | Water | Ad 100 | Ad 100 |
| Plantacare PS 10 | Sodium Laureth Sulfate, Lauryl Glucoside | 20,0 | 20,0 |
| Euperlan PK 771 | Glycol Distearate, Sodium Lauryl Sulfate, Cocamide MEA, Laureth-10 | 6,0 | 6,0 |
| Dragocid Liquid | Phenoxyethanol, Methylparaben, Ethylparaben, Butylparaben, Propylparaben, Isobutylparaben | 0,5 | 0,5 |
| Natrium Chlorid | Sodium Chloride | 1,4 | 1,4 |
| Citronensäure Monohydrat kristallin | Citric Acid | 0,1 | 0,1 |
| Parfumöl gemäß P1, P2, P3, P4 oder P5 | Parfum (Fragrance) | 0,5 | 0,8 |

### Beispiel F4 - Duschgel

| **Material** | **INCI-Name** | **Gew.-%** | **Gew.-%** |
|---|---|---|---|
| Entionisiertes Wasser | Wasser | Ad 100 | Ad 100 |
| Plantacare PS 10 | Sodium Laureth Sulfate, Lauryl Glucoside | 20,0 | 20,0 |
| Dragocid Liquid | Phenoxyethanol, Methylparaben, Ethylparaben, Butylparaben, Propylparaben, Isobutylparaben | 0,5 | 0,5 |
| Natriumchlorid | Sodium Chloride | 1,4 | 1,4 |
| Citronensäure Monohydrat kristallin | Citric Acid | 1,3 | 1,3 |
| Parfumöl gemäß P1, P2, P3, P4 oder P5 | Parfum (Fragrance) | 0,5 | 0,7 |

### Beispiel F5 - Weichspüler

| **Material** | **Chemischer Name** | **Funktion** | **Gew.-%** | **Gew.-%** |
|---|---|---|---|---|
| Entionisiertes Wasser | Wasser | Lösungsmittel | Ad 100 | Ad 100 |
| Rewoquat WE 18 | Dialkylesterammoniumethosulfate | Kationisches Tensid | 16,6 | 16,6 |
| Mergal K9N | 5-Chloro-2-methyl-3-(2H)-isothiazolone und 2-methyl-3-(2H)-isothiazolone | Konservierungsmittel | 0,10 | 0,10 |
| Dow Corning 1520 Antifoam | Polydimethyl-siloxane | Entschäumer | 0,30 | 0,30 |
| Magnesium Chlorid 1%ige Lösung | Magnesium Chlorid Lösung | Konsistenzgeber | 10,00 | 10,00 |
| Parfumöl gemäß P1, P2, P3, P4 oder P5 | | Fragrance | 0,55 | 0,75 |

### Beispiel F6 - Eau de Cologne / Eau de Toilette

| **Inhaltsstoffe** | **Gew.-%** | **Gew.-%** |
|---|---|---|
| Parfumöl gemäß P1, P2, P3, P4 oder P5 | 5 | 10 |
| Ethanol | Ad 100 | Ad 100 |
| Wasser | 18 | 10 |

### Beispiel F7 - Aerosol-Pumpspray

| **Inhaltsstoffe** | **Gew.-%** | **Gew.-%** |
|---|---|---|
| Parfumöl gemäß P1, P2, P3, P4 oder P5 | 1,0 | 1,5 |
| Ethanol | Ad 100 | Ad 100 |
| Wasser | 5,0 | 8,0 |
| Alpha-Tocopherol | 0,20 | 0,20 |
| Hydroxypropylcellulose | 0,20 | - |
| Rosmarinextrakt, in Ethanol löslich | 0,22 | - |
| Cetylalkohol | 1,00 | 0,50 |

### Beispiel F8 - Shampoo

| **Inhaltsstoffe** | **Gew.-%** | **Gew.-%** | **Gew.-%** |
|---|---|---|---|
| Natriumlaurylethersulfat (z.B. Texapon NSO, Fa. Cognis Deutschland GmbH) | 12 | 12 | 12 |
| Cocamidopropylbetain (z.B. Dehyton K, Fa. Cognis Deutschland GmbH) | 2 | 2 | 2 |
| Natriumchlorid | 1,4 | 1,4 | 1,4 |
| Citronensäure | 1,3 | 1,3 | 1,3 |
| Phenoxyethanol, Methyl-, Ethyl-, Butyl- und Propylparaben | 0,5 | 0,5 | 0,5 |
| Parfumöl gemäß P1, P2, P3, P4 oder P5 | 0,3 | 0,5 | 0,7 |
| Wasser | Ad 100 | Ad 100 | Ad 100 |

### Beispiel F9 - Waschpulver

| **Inhaltsstoffe** | **Gew.-%** | **Gew.-%** | **Gew.-%** |
|---|---|---|---|
| Lineares Na-Alkylbenzolsulfonat | 8,8 | 8,8 | 8,8 |
| Ethoxylierter Fettalkohol C12-18 (7 EO) | 4,7 | 4,7 | 4,7 |
| Na-Seife | 3,2 | 3,2 | 3,2 |
| Entschäumer DOW CORNING(R) 2-4248S POWDERED ANTIFOAM, Silikonöl auf Zeolith als Trägermaterial | 3,9 | 3,9 | 3,9 |
| Zeolith 4A | Ad 100 | Ad 100 | Ad 100 |
| Na-Carbonat | 11,6 | 11,6 | 11,6 |
| Na-Salz eines Copolymers aus Acryl- und Maleinsäure (Sokalan CP5) | 2,4 | 2,4 | 2,4 |
| Na-Silicat | 3,0 | 3,0 | 3,0 |
| Carboxymethylcellulose | 1,2 | 1,2 | 1,2 |
| Dequest | 2,8 | 2,8 | 2,8 |
| 2066([[(Phosphonomethyl)imino]bis[(ethylennitrilo)bis (methylen)]]tetrakisphosphonsäure, Natriumsalz) | | | |
| Optischer Aufheller | 0,2 | 0,2 | 0,2 |
| Na-Sulfat | 6,5 | 6,5 | 6,5 |
| Protease | 0,4 | 0,4 | 0,4 |
| Natriumperborat tetrahydrat | 21,7 | 21,7 | 21,7 |
| Parfumöl gemäß P1, P2, P3, P4 oder P5 | 0,25 | 0,35 | 0,5 |
| EDTA | 1,0 | 1,0 | 1,0 |

### Beispiel F10 - Flüssigwaschmittel

| **Inhaltsstoffe** | **Gew.-%** |
|---|---|
| Entionisiertes Wasser | 39,9 |
| Optischer Aufheller | 0,10 |
| Kokos Fettsäuren (C12-C18) | 7,5 |
| Kaliumhydroxid 50%ige Lösung 22 | 4,3 |
| Propan-1,2-diol | 5,00 |
| Fettalkohole C12-C15, 8 EO | 12,0 |
| Na-Salz sekundärer Alkylsulfonate (C13-C17) | 17,0 |
| Triethanolamin | 2,0 |
| Trinatriumcitrat Dihydrat | 5,0 |
| Dequest 2066([[(Phosphonomethyl)imino]bis[(ethylennitrilo)bis (methylen)]]tetrakis-phosphonsäure, Natriumsalz) | 3,0 |
| Ethanol | 3,0 |
| Enzyme | 0,7 |
| Parfumöl gemäß P1, P2, P3, P4 oder P5 | 0,5 |

### Beispiel F11 - Flüssigwaschmittel Konzentrat

| **Inhaltsstoffe** | **Gew.-%** |
|---|---|
| Entionisiertes Wasser | 13,4 |
| Kokos Fettsäuren (C12-C18) | 10,0 |
| Fettalkohole C12-C15, 8 EO | 26,0 |
| Na-Salz sekundärer Alkylsulfonate (C13-C17) | 26,5 |
| Triethanolamin | 8,5 |
| Na-Salz von Fettalkoholsulfaten C12-C14 | 3,0 |
| Ethanol | 5,5 |
| Harnstoff | 4,5 |
| Enzyme | 0,9 |
| Citronensäure | 1,0 |
| Parfumöl gemäß P1, P2, P3, P4 oder P5 | 0,7 |

## Patentansprüche

1. Verwendung
einer einzelnen Verbindung der Formel (I) oder einer Mischung umfassend oder bestehend aus zwei oder mehr Verbindungen der Formel (I) 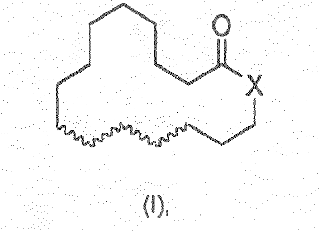
wobei für die Verbindung der Formel (I) bzw. jede Verbindung der Formel (I) gilt, dass keine, eine oder zwei der vier gewellten Linien eine Doppelbindung bedeutet bzw. bedeuten und die übrigen gewellten Linien jeweils eine Einfachbindung bedeuten und X ausgewählt ist aus -O-, -CH2- und -O-CH2-, vorzugsweise aus -O- und - CH2-, zum Verstärken des bzw. eines oder mehrerer angenehmer Geruchseindrücke eines oder mehrerer angenehm riechender Stoffe, wobei der/die Stoff(e) keine Verbindung der Formel (I) ist bzw. sind,
wobei (i) die Verbindung der Formel (I) bzw. (ii) eine, mehrere oder sämtliche Verbindungen der Formel (I) ausgewählt ist bzw. sind aus der Gruppe bestehend aus Cyclohexadec-8-en-1-on (Aurelione, CAS No. 88642-03-9, 3100-36-5; Globanone, CAS No. 3100-36-5) und Cyclohexadecanon (Isomuscone, CAS No. 2550-52-9),
und wobei der bzw. einer, mehrere oder sämtliche der angenehm riechenden, nicht der Formel (I) entsprechende(n) Stoff(e) ausgewählt ist bzw. sind aus der Gruppe bestehend aus Methyldihydrojasmonat, Benzylsalicylat, cis-3-Hexenylsalicylat, Isoamylsalicylat, Hexylsalicylat, 2,3,8,8-Tetramethyl-1,2,3,4,5,6,7,8-octahydro-2-naphthalenylmethylketon, Linalylacetat, Ethyllinalylacetat, Cedrylmethylether, Cedrylmethylketon, Cedrylacetat, (4aR,5R,7aS,9R)-Octahydro-2,2,5,8,8,9a-hexamethyl-4H-4a,9-methano azuleno(5,6-d)-1,3-dioxol), 1',1',5',5'-Tetra methyl-hexahydro-spiro[1.3-dioxolan-2.8'(5'H)-2H-2.4a]methanonaphthalin, Cyclododecylmethylether, (Ethoxymethoxy)cyclododecan, Decahydro-beta-naphthylacetat, 4,7-Methano-3a,4,5,6,7,7a-hexahydro-5(-6)-indenylacetat, Allyl-3-cyclohexylpropionat, Allylcyclohexyloxyacetat, Benzylbenzoat, Benzylcinnamat, 15-Hydroxy-Pentadecanonsäurelacton, 3-Methyl-cyclopentadecenon, 1,3,4,6,7,8-hexahydro-4,6,6,7,8,8-hexamethyl Cyclopenta[g]-2-benzopyran, 2-[1-(3,3-dimethylcyclohexyl)ethoxy]-2-methyl-,1-propanoat, 1,4-Dioxacycloheptadecan-5,17-dion, 3-Methy-cyclopentadecanon, 3a,6,6,9a-Tetramethyl dodecahydronaphtho[2,1-b]furan, alpha-Irone, beta-Irone, alpha-n-Methylionon, beta-n-Methylionon, alpha-Isomethylionon, beta-Isomethylionon und Allylionon, 2-Methyl-3-(4-tert-butylphenyl)propanal, 4-(4-Hydroxy-4-methylpentyl)-3-cyclohexencarboxaldehyd, 2-Methyl-4-(2,6,6-trimethyl-1-cyclohexen-1-yl)-2-butenal, 1-Methyl-4-(4-methyl-3-penten-1-yl)-3-cyclohexencarbox aldehyd, 3-(3-lsopropyl-phenyl)-butyraldehyd, (E)-2,6,10-Trimethyl-undeca-5,9-dienal, Benzo[1,3]dioxole-5-carbaldehyd, 2,2-Dimethyl-3-phenyl-propan-1-ol, 2,2-Dimethyl-3-m-tolyl-propan-1-ol, 1-(4-Isopropyl-cyclohexyl)-ethanol, (4-Isopropyl-cyclohexyl)-methanol, 2-Phenyl-ethanol, 2-Isobutyl-4-methyl-tetrahydropyran-4-ol, 3,7-Dimethyl-octa-1,6-dien-3-ol, (Z)-3,7-Dimethyl-octa-2,6-dien-1-ol, (E)-3,7-Dimethyl-octa-2,6-dien-1-ol, 3,7-Dimethyl-oct-6-en-1-ol, 2,6-Dimethyl-oct-7-en-2-ol, 3,7-Dimethyl-octan-1-ol, 2-Methyl-6-methylenoct-7-en-2-ol und (E/Z)-3,7-Dimethyl-nona-1,6-dien-3-ol.

2. Verwendung nach Anspruch 1, zum Verstärken des bzw. eines oder mehrerer angenehmer Geruchseindrücke eines oder mehrerer angenehm riechender Stoffe, wobei der/die Stoff(e) keine Verbindung der Formel (I) ist bzw. sind, nämlich zum Verstärken bzw. Verbessern der natürlichen Frische und/oder Ausstrahlung eines oder mehrerer angenehm riechender Stoffe und/oder zum Verstärken der bzw. einer blumigen Geruchsnote eines oder mehrerer angenehm riechender Stoffe, insbesondere einer Geruchsnote des Typs Jasmin.

3. Verwendung nach einem der vorangehenden Ansprüche, wobei der bzw. einer, mehrere oder sämtliche der angenehm riechenden, nicht der Formel (I) entsprechende(n) Stoff(e) ausgewählt ist bzw. sind aus der Gruppe bestehend aus Riechstoffen mit einer Molmasse im Bereich von 150 g/mol bis 285 g/mol, insbesondere Alkoholen, Aldehyden, Ketonen, Ethern, Estern und Carboxylaten mit einer Molmasse im Bereich von 150 g/mol bis 285 g/mol.

4. Verwendung nach einem der vorangehenden Ansprüche, wobei das Verhältnis der Gesamtmasse an angenehm riechenden, nicht der Formel (I) entsprechenden Stoffen zur Gesamtmasse an Verbindung(en) der Formel (I) größer oder gleich 99 : 1 ist, bevorzugt größer oder gleich 99,9 : 0,1, besonders bevorzugt größer oder gleich 99,999 : 0,001, und/oder
die zum Verstärken eingesetzte Menge an Verbindung(en) der Formel (I) nicht ausreicht, um einen Moschusgeruch zu vermitteln.

5. Riechstoffmischung, vorzugsweise Parfümöl, umfassend oder bestehend aus
(A) (i) einer einzelnen Verbindung der Formel (I) oder (ii) einer Mischung umfassend oder bestehend aus zwei oder mehr Verbindungen der Formel (I) 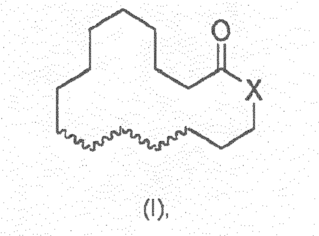
wobei für die Verbindung der Formel (I) bzw. jede Verbindung der Formel (I) gilt, dass
keine, eine oder zwei der vier gewellten Linien eine Doppelbindung bedeutet bzw. bedeuten und die übrigen gewellten Linien jeweils eine Einfachbindung bedeuten und
X ausgewählt ist aus -O-, -CH2- und -O-CH2-, vorzugsweise aus -O- und -CH2-,und
(B) einem, zwei, drei, vier, fünf, sechs oder mehr (weiteren) Riechstoffen wobei der bzw. die (weitere(n)) Riechstoff(e) keine Verbindung der Formel (I) ist bzw. sind und einer, zwei, drei, vier, fünf, sechs oder mehr bzw. sämtliche der (weiteren) Riechstoffe eine Molmasse im Bereich von 150 g/mol bis 285 g/mol besitzt bzw.
besitzen, **dadurch gekennzeichnet, dass** das Verhältnis der Gesamtmasse an nicht der Formel (I) entsprechenden Riechstoffen zur Gesamtmasse an Verbindung(en) der Formel (I) größer oder gleich 99,9 : 0,1 ist, besonders bevorzugt größer oder gleich 99,999 : 0,001,
wobei (i) die Verbindung der Formel (I) bzw. (ii) eine, mehrere oder sämtliche Verbindungen der Formel (I) ausgewählt ist bzw. sind aus der Gruppe bestehend aus Cyclohexadec-8-en-1-on (Aurelione, CAS No. 88642-03-9, 3100-36-5; Globanone, CAS No. 3100-36-5) und Cyclohexadecanon (Isomuscone, CAS No. 2550-52-9),)
und wobei der Riechstoff bzw. einer, mehrere oder sämtliche Riechstoffe gemäß Bestandteil (B) ausgewählt ist bzw. sind aus der Gruppe bestehend aus Methyldihydrojasmonat, Benzylsalicylat, cis-3-Hexenylsalicylat, Isoamylsalicylat, Hexylsalicylat, 2,3,8,8-Tetramethyl-1,2,3,4,5,6,7,8-octahydro-2-naphthalenylmethylketon, Linalylacetat, Ethyllinalylacetat, Cedrylmethylether, Cedrylmethylketon, Cedrylacetat, (4aR,5R,7aS,9R)-Octahydro-2,2,5,8,8,9a-hexamethyl-4H-4a,9-methano azuleno(5,6-d)-1,3-dioxol), 1',1',5',5'-Tetra methyl-hexahydro-spiro[1.3-dioxolan-2.8'(5'H)-2H-2.4a]methanonaphthalin, Cyclododecylmethylether, (Ethoxymethoxy)cyclododecan, Decahydro-beta-naphthylacetat, 4,7-Methano-3a,4,5,6,7,7a-hexahydro-5(-6)-indenylacetat, Allyl-3-cyclohexylpropionat, Allylcyclohexyloxyacetat, Benzylbenzoat, Benzylcinnamat, 15-Hydroxy-Pentadecanonsäurelacton, 3-Methyl-cyclopentadecenon, 1,3,4,6,7,8-hexahydro-4,6,6,7,8,8-hexamethyl Cyclopenta[g]-2-benzopyran, 2-[1-(3,3-dimethylcyclohexyl)ethoxy]-2-methyl-,1-propanoat, 1,4-Dioxacycloheptadecan-5,17-dion, 3-Methy-cyclopentadecanon, 3a,6,6,9a-Tetramethyl dodecahydronaphtho[2,1-b]furan, alpha-Irone, beta-trone, alpha-n-Methylionon, beta-n-Methylionon, alpha-Isomethylionon, beta-Isomethylionon und Allylionon, 2-Methyl-3-(4-tert-butylphenyl)propanal, 4-(4-Hydroxy-4-methylpentyl)-3-cyclohexencarboxaldehyd, 2-Methyl-4-(2,6,6-trimethyl-1-cyclohexen-1-yl)-2-butenal, 1-Methyl-4-(4-methyl-3-penten-1-yl)-3-cyclohexencarbox aldehyd, 3-(3-Isopropyl-phenyl)-butyraldehyd, (E)-2,6,10-Trimethyl-undeca-5,9-dienal, Benzo[1,3]dioxole-5-carbaldehyd, 2,2-Dimethyl-3-phenyl-propan-1-ol, 2,2-Dimethyl-3-m-tolyl-propan-1-ol, 1-(4-Isopropyl-cyclohexyl)-ethanol, (4-Isopropyl-cyclohexyl)-methanol, 2-Phenyl-ethanol, 2-Isobutyl-4-methyl-tetrahydro-pyran-4-ol, 3,7-Dimethyl-octa-1,6-dien-3-ol, (Z)-3,7-Dimethyl-octa-2,6-dien-1-ol, (E)-3,7-Dimethyl-octa-2,6-dien-1-ol, 3,7-Dimethyl-oct-6-en-1-ol, 2,6-Dimethyl-oct-7-en-2-ol, 3,7-Dimethyl-octan-1-ol, 2-Methyl-6-methylenoct-7-en-2-ol und (E/Z)-3,7-Dimethyl-nona-1,6-dien-3-ol.

6. Riechstoffmischung nach Anspruch 5, wobei die Menge an Verbindung(en) der Formel (I) in der Riechstoffmischung ausreicht, um den bzw. einen oder mehrere angenehme Geruchseindrücke des bzw. eines, mehrerer oder sämtlicher Riechstoffe gemäß Bestandteil (B) zu verstärken, insbesondere um die natürliche Frische und/oder Ausstrahlung des bzw. eines, mehrerer oder sämtlicher Riechstoffe gemäß Bestandteil (B) zu verstärken bzw. zu verbessern und/oder um die bzw. eine blumige Geruchsnote des bzw. eines, mehrerer oder sämtlicher Riechstoffe gemäß Bestandteil (B), vorzugsweise eine Geruchsnote des Typs Jasmin, zu verstärken.

7. Riechstoffmischung nach einem der Ansprüche 5 bis 6, wobei der Anteil an Verbindung(en) der Formel (I) bezogen auf das Gesamtgewicht der Riechstoffmischung 0,1 Gew.-% oder weniger beträgt, besonders bevorzugt 0,001 Gew.-% oder weniger.

8. Parfümiertes Produkt, enthaltend eine Riechstoffmischung nach einem der Ansprüche 5 bis 7, vorzugsweise ein Parfümöl nach einem der Ansprüche 5 bis 7, in einer sensorisch wirksamen Menge, wobei der Anteil der Riechstoffmischung bezogen auf das Gesamtgewicht des Produkts im Bereich von 0,01 bis 10 Gew.-%, bevorzugt im Bereich von 0,1 bis 5 Gew.-%, besonders bevorzugt im Bereich von 0,25 bis 3 Gew.-%, liegt.

9. Parfümiertes Produkt nach Anspruch 8, wobei das Produkt ausgewählt ist aus der Gruppe bestehend aus Parfüm-Extraits, Eau de Parfums, Eau de Toilettes, Rasierwässer, Eau de Colognes, Pre-shave-Produkte, Splash-Colognes, parfümierte Erfrischungstücher, saure, alkalische und neutrale Reinigungsmittel, Textilerfrischer, Bügelhilfen, flüssige Waschmittel, pulverförmige Waschmittel, Wäschevorbehandlungsmittel, Wäscheweichspüler, Waschseifen, Waschtabletten, Desinfektionsmittel, Oberflächendesinfektionsmittel, Luftverbesserer, Aerosolsprays, Wachse und Polituren, Körperpflegemittel, Handcremes und -lotionen, Fußcremes und -lotionen, Enthaarungscremes und -lotionen, After-shave-Cremes und -lotionen, Bräunungscremes und -lotionen, Haarpflegeprodukten, Deodorantien und Antiperspirantien, Produkte der dekorativen Kosmetik, Kerzen, Lampenöle, Räucherstäbchen, Insektizide, Repellentien und Treibstoffe.

10. Verfahren zum Herstellen eines parfümierten Produkts, insbesondere eines parfümierten Produkts nach einem der Ansprüche 8 oder 9, umfassend folgende Schritte:
(i) Bereitstellen der Bestandteile (A) und (B) wie in einem der Ansprüche 5 bis 7 definiert, vorzugsweise Bereitstellen einer Riechstoffmischung nach einem der Ansprüche 5 bis 7,
(ii) Bereitstellen eines oder mehrerer weiterer Bestandteile des herzustellenden parfümierten Produkts, und
(iii) Inkontaktbringen oder Mischen der in Schritt ii) bereitgestellten weiteren Bestandteile mit einer sensorisch wirksamen Menge der in Schritt i) bereitgestellten Bestandteile (A) und (B) bzw. der in Schritt i) bereitgestellten Riechstoffmischung.

## Claims

1. Use of
a single compound of formula (I) or
a mixture comprising or consisting of two or more compounds of formula (I) 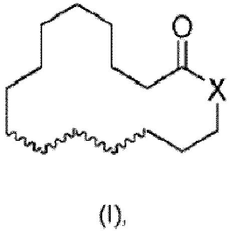
wherein for said compound (I) and each compound of formula (I) respectively applies, that none, one or two of the waved lines stand for a double bond and respectively applies that the remaining waved lines stand for single bonds, and
X is chosen from -O-, -CH2- und -OCH2-, preferably from -O- and -CH2-,
for augmenting of one or more pleasant olfactory impression of one or more pleasant smelling compounds, whereby this compound/these compounds is/are different from compounds of formula (I),
wherein
(i) said compound of formula (I) or respectively
(ii) one, more or all compounds of formula (I)
are selected from the group consisting of cyclohexadec-8-en-1-one (Aurelione, CAS No. 88642-03-9, 3100-36-5; Globanone, CAS No 3100-35-5) and cyclohexadecanone (Iso-muscone, CAS No. 2550-52-9),
and wherein said one, more or all pleasant smelling compounds different from formula (I) are selected from the group consisting of methyldihydrojasmonate, benzylsalicylate, cis-3-hexenylsalicylate, isoamylsalicylate, hexylsalicylate, 2,3,8,8-tetramethyl-1,2,3,4,5,6,7,8-octahydro-2-naphthalenylmethyl¬ke¬tone, linalylacetate, ethyllinalylacetate, cedrylmethylether, cedrylmethylketone, cedryl¬acetate, (4aR,5R,7aS,9R)-octahydro-2,2,5,8,8,9a-hexamethyl-4H-4a,9-methanoazu¬leno¬(5,6-d)-1,3-dioxol), 1',1',5',5'-Tetra methyl-hexahydro-spiro[1.3-dioxolan-2.8'(5'H)-2H-2.4a]methanonaphthaline, cyclododecylmethylether, (ethoxymethoxy)cyclo¬idodecane, decahydro-beta-naphthylacetate, 4,7-methano-3a,4,5,6,7,7a-hexahydro-5(-6)-indenyl-acetate, allyl-3-cyclohexylpropionate, allylcyclohexyloxyacetate, benzylbenzoate, benzylcinnamate, 15-hydroxy-pentadecanonsäurelactone, 3-methyl-cyclopenta-decenone, 1,3,4,6,7,8-hexa¬hydro-4,6,6,7,8,8-hexamethyl cyclopenta[g]-2-benzopyrane, 2-[1-(3,3-dimethylcyclohexyl)ethoxy]-2-methyl-,1-propanoate, 1,4-dioxacyclohepta-decane-5,17-dion, 3-methy-cyclopentadecanone, 3a,6,6,9a-Tetramethyldodecahydronaphtho[2,1-b]furane, alpha-lrone, beta-lrone, alpha-n-methylionone, beta-n-methylionone, alpha-Isomethylionone, beta-Isomethylionone and allylionone, 2-methyl-3-(4-tert-butylphenyl)propanal, 4-(4-hydroxy-4-methylpentyl)-3-cyclohexencarboxaldehyde, 2-methyl-4-(2,6,6-trimethyl-1-cyclohexen-1-yl)-2-butenal, 1-methyl-4-(4-methyl-3-penten-1-yl)-3-cyclohexencarboxaldehyde, 3-(3-Isopropyl-phenyl)-butyraldehyde, (E)-2,6,10-trimethyl-undeca-5,9-dienal, benzo[1,3]dioxole-5-carbaldehyde, 2,2-dimethyl-3-phenyl-propan-1-ol, 2,2-Dimethyl-3-m-tolyl-propan-1-ol, 1-(4-Isopropyl-cyclohexyl)-ethanol, (4-lsopropyl-cyclohexyl)-methanol, 2-phenyl-ethanol, 2-Isobutyl-4-methyl-tetrahydro-pyran-4-ol, 3,7-dimethyl-octa-1,6-dien-3-ol, (Z)-3,7-dimethyl-octa-2,6-dien-1-ol, (E)-3,7-dimethyl-octa-2,6-dien-1-ol, 3,7-dimethyl-oct-6-en-1-ol, 2,6-dimethyl-oct-7-en-2-ol, 3,7-dimethyl-octan-1-ol, 2-methyl-6-methylenoct-7-en-2-ol and (E/Z)-3,7-dimethyl-nona-1,6-dien-3-ol.

2. The use according to claim 1, for augmentation of said one or more pleasant olfactory impression of one or more pleasant smelling compounds, wherein said compound/s does/do not match with the compounds of formula (I), namely for augmentation or respectively improvement of natural freshness and/or charisma and/or improvement of a floral olfactory note of one or more pleasant smelling compounds, particularly of an olfactory note of jasmine type.

3. The use according to any of the preceding claims, wherein said one, more or all pleasant smelling compounds different from formula (I) are selected from the group consisting of fragrances having a molar mass within the range of from 150 g/mol to 285 g/mol, par-ticularly alcohols, aldehydes, ketones, ethers, esters and carboxylates having a molar mass in the range of from 150 g/mol to 285 g/mol.

4. The use according to any of the preceding claims, wherein the ratio of total mass of the pleasant tasting compound different from formula (I) to the total mass of compound(s) of formula (I) exceeds or is equal to 99:1, preferably exceeds or is equal 99.9:0.1, and more preferably exceeds or is equal to 99.999:0.001, and or
the amount of compound(s) used for improvement is not sufficient for providing a musk odour.

5. A fragrance mixture, preferably a perfume oil, comprising or consisting of
(A) (i) a single compound of formula (I) or (ii) a mixture comprising or consisting of two or more compounds of formula (I) 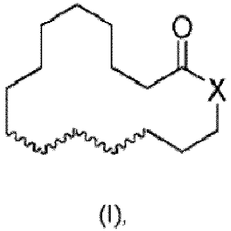
wherein for said compound (I) and each compound of formula (I) respectively applies, that none, one or two of the waved lines stands for a double bond and respectively applies that the remaining waved lines stand for single bonds, and
X is chosen from -O-, -CH2- und -OCH2-, preferably from -O- and -CH2-, and
(B) one, two, three, four, five, six or more (additional) fragrances different from formula (I), and one, two, three, four, five, six or more or all of said (additional) fragrances have a molar mass in the range of 150 g/mol to 285 g/mol, **characterized in that** the ratio of total mass of the pleasant tasting compound different from formula (I) to the total mass of compound(s) of formula (I) exceeds or is equal to 99.9:0.1, and more preferably exceeds or is equal to 99.999:0.001,
wherein (i) said compound of formula (I) or respectively (ii) one, more or all compounds of formula (I) are selected from the group consisting of cyclohexadec-8-en-1-one (Aurelione, CAS No. 88642-03-9, 3100-36-5; Globanone, CAS No 3100-35-5) and cyclohexadecanone (Iso-muscone, CAS No. 2550-52-9),
and wherein said fragrance or one, more or all fragrances according to component (B) are selected from the group consisting of methyldihydrojasmonate, benzylsalicylate, cis-3-hexenylsalicylate, isoamylsalicylate, hexylsalicylate, 2,3,8,8-tetramethyl-1,2,3,4,5,6,7,8-octahydro-2-naphthalenylmethyl¬ke¬tone, linalylacetate, ethyllinalylacetate, cedrylmethylether, cedrylmethylketone, cedryl¬acetate, (4aR,5R,7aS,9R)-octahydro-2,2,5,8,8,9a-hexamethyl-4H-4a,9-methanoazu¬leno¬(5,6-d)-1,3-dioxol), 1',1',5',5'-Tetra methyl-hexahydro-spiro[1.3-dioxolan-2.8'(5'H)-2H-2.4a]methanonaphthaline, cyclododecylmethylether, (ethoxymethoxy)cyclo¬dodecane, decahydro-beta-naphthylacetate, 4,7-methano-3a,4,5,6,7,7a-hexahydro-5(-6)-indenyl¬acetate, allyl-3-cyclohexylpropionate, allylcyclohexyloxyacetate, benzylbenzoate, benzyl¬cinna¬mate, 15-hydroxy-pentadecanonsäurelactone, 3-methyl-cyclopenta¬decenone, 1,3,4,6,7,8-hexa¬hydro-4,6,6,7,8,8-hexamethyl cyclopenta[g]-2-benzopyrane, 2-[1-(3,3-dimethylcyclohexyl)ethoxy]-2-methyl-,1-propanoate, 1,4-dioxacyclohepta¬decane-5,17-dion, 3-methy-cyclopentadecanone, 3a,6,6,9a-Tetramethyldodecahydronaphtho[2,1-b]furane, alpha-lrone, beta-lrone, alpha-n-methylionone, beta-n-methylionone, alpha-Isomethylionone, beta-Isomethylionone and allylionone, 2-methyl-3-(4-tert-butylphenyl)propanal, 4-(4-hydroxy-4-methylpentyl)-3-cyclohexencarboxaldehyde, 2-methyl-4-(2,6,6-trimethyl-1-cyclohexen-1-yl)-2-butenal, 1-methyl-4-(4-methyl-3-penten-1-yl)-3-cyclohexencarboxaldehyde, 3-(3-Isopropyl-phenyl)-butyraldehyde, (E)-2,6,10-trimethyl-undeca-5,9-dienal, benzo[1,3]dioxole-5-carbaldehyde, 2,2-dimethyl-3-phenyl-propan-1-ol, 2,2-Dimethyl-3-m-tolyl-propan-1-ol, 1-(4-lsopropyl-cyclohexyl)-ethanol, (4-lsopropyl-cyclohexyl)-methanol, 2-phenyl-ethanol, 2-Isobutyl-4-methyl-tetrahydro-pyran-4-ol, 3,7-dimethyl-octa-1,6-dien-3-ol, (Z)-3,7-dimethyl-octa-2,6-dien-1-ol, (E)-3,7-dimethyl-octa-2,6-dien-1-ol, 3,7-dimethyl-oct-6-en-1-ol, 2,6-dimethyl-oct-7-en-2-ol, 3,7-dimethyl-octan-1-ol, 2-methyl-6-methylenoct-7-en-2-ol and (E/Z)-3,7-dimethyl-nona-1,6-dien-3-ol.

6. The fragrance mixture of claim 5, wherein the amount of compound(s) of formula (I) in the mixture is sufficient to augment one or more pleasant olfactory impression of one, more or all fragrances according to component (B), particularly for augmenting or improving natural freshness and/or charisma of one, more or all fragrances according to component (B), and/or augmenting a floral note of one, more or all fragrance according to component (B), particularly an olfactory impression of jasmine type.

7. The fragrance mixture of any of claims 5 to 6, wherein the amount of compound(s) of formula (I) calculated on the total mass of said fragrance mixture is 0.1 wt.-% or less, and more preferably 0.001 wt.-% or less.

8. A perfumed product comprising a fragrance mixture according to claims 5 to 7, preferably a perfume oil according to claims 5 to 7, in a sensorial working amount, wherein the amount of said fragrance mixture calculated on the total mass of the product ranges from 0.01 to 10 wt.-%, more preferably from 0.1 to 5 wt.-%, and most preferably from 0.25 to 3 wt.-%.

9. The perfumed product of claim 8, wherein said product is selected from the group consisting of perfume extracts, eau de perfumes, eau de toilettes, after shaves, eau de colognes, pre-shave products, splash colognes, perfumed freshening wipes, acidic, alkaline or neutral cleaning agents, textile fresheners, ironing aids, liquid detergents, powder detergents, textile pre-treatment agents, textile softeners, detergent soaps, detergent tablets, disinfecting agents, surface disinfecting agents, air fresheners, aerosol sprays, waxes and polishes, personal care agents, hand crèmes and lotions, foot crèmes and lotions, after-shave crèmes and lotions, tanning crèmes and lotions, hair care agents, deodorants and antiperspirants, products for decorative cosmetics, candles, lamp oils, joss sticks, insecticides, repellents and fuels.

10. A process for producing a perfumed product, particularly a perfumed product according to claims 8 or 9, encompassing the following steps:
(i) providing components (A) and (B) as defined in claims 5 to 7, preferably providing a fragrance mixture according to claims 5 to 7;
(ii) providing one or more additional components of the perfumed product to be produced; and
(iii) bringing in contact or mixing of the additional components provided in step (li) with a sensorial working amount of components (A) and (B) provided in step (i) or of the fragrance mixture provided in step (i).

## Revendications

1. Utilisation d'un seul composé de formule (I) ou d'un mélange comprenant ou constitué par deux composés, ou plus, de formule (I) 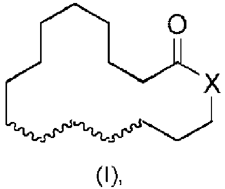
où, pour le composé de formule (I) ou pour chaque composé de formule (I)
aucune, une ou deux des quatre lignes ondulées signifie ou signifient une double liaison et les autres lignes ondulées signifient à chaque fois une simple liaison et X est choisi parmi -O-, -CH₂- et -O-CH₂-, de préférence parmi -O- et -CH₂-,
pour le renforcement de la ou d'une ou de plusieurs impression(s) d'odeur agréable d'une ou de plusieurs substance(s) à odeur agréable, la/les substance(s) n'étant pas un composé de formule (I),
(i) le composé de formule (I) ou (ii) un, plusieurs ou tous les composé(s) de formule (I) étant choisi(s) dans le groupe constitué par la cyclohexadéc-8-én-1-one (Aurelione, n° CAS 88642-03-9, 3100-36-5 ; Globanone, n° CAS 3100-36-5) et la cyclohexadécanone (Isomuscone, n° CAS 2550-52-9),
et la ou une, plusieurs ou l'ensemble des substance(s) à odeur agréable, ne correspondant pas à la formule (I), étant choisie(s) dans le groupe constitué par le dihydrojasmonate de méthyle, le salicylate de benzyle, le salicylate de cis-3-hexényle, le salicylate d'isoamyle, le salicylate d'hexyle, la 2,3,8,8-tétraméthyl-1,2,3,4,5,6,7,8-octa-hydro-2-naphtalénylméthylcétone, l'acétate de linalyle, l'acétate d'éthyllinalyle, le cédrylméthyléther, la cédrylméthylcétone, l'acétate de cédryle, le (4aR,5R,7aS,9R)-octahydro-2,2,5,8,8,9a-hexaméthyl-4H-4a,9-méthano-azuléno(5,6-d)-1,3-dioxole, le 1',1',5',5'-tétraméthyl-hexahydrospiro[1,3-dioxolane-2,8'(5'H)-2H-2,4a]méthanonaphtalène, le cyclododécylméthyléther, l'(éthoxyméthoxy)cyclododécane, l'acétate de décahydro-bêta-naphtyle, l'acétate de 4,7-méthano-3a,4,5,6,7,7a-hexahydro-5(-6)-indényle, le propionate d'allyl-3-cyclohexyle, le cyclohexyloxyacétate d'allyle, le benzoate de benzyle, le cinnamate de benzyle, la lactone de l'acide 15-hydroxy-pentadécanoïque, la 3-méthylcyclo-pentadécénone, le 1,3,4,6,7,8-hexahydro-4,6,6,7,8,8-hexaméthylcyclopenta[g]-2-benzopyrane, le 1-propanoate de 2-[1-(3,3-diméthylcyclohexyl)éthoxy]-2-méthyle, la 1,4-dioxacycloheptadécane-5,17-dione, la 3-méthycyclopentadécanone, le 3a,6,6,9a-tétraméthyldodécahydronaphto[2,1-b]furanne, l'alpha-irone, la bêta-irone, l'alpha-n-méthylionone, la bêta-n-méthylionone, l'alpha-isométhylionone, la bêta-isométhylionone et l'allylionone, le 2-méthyl-3-(4-tert-butylphényl)propanal, le 4-(4-hydroxy-4-méthylpentyl)-3-cyclohexènecarboxaldéhyde, le 2-méthyl-4-(2,6,6-triméthyl-1-cyclohexén-1-yl)-2-buténal, le 1-méthyl-4-(4-méthyl-3-pentén-1-yl)-3-cyclohexènecarboxaldéhyde, le 3-(3-isopropylphényl)-butyraldéhyde, le (E)-2,6,10-triméthyl-undéca-5,9-diénal, le benzo[1,3]dioxole-5-carbaldéhyde, le 2,2-diméthyl-3-phénylpropan-1-ol, le 2,2-diméthyl-3-m-tolylpropan-1-ol, le 1-(4-isopropylcyclohexyl)-éthanol, le (4-isopropylcyclohexyl)-méthanol, le 2-phényléthanol, le 2-isobutyl-4-méthyltétrahydropyran-4-ol, le 3,7-diméthylocta-1,6-dién-3-ol, le (Z)-3,7-diméthylocta-2,6-dién-1-ol, le (E)-3,7-diméthylocta-2,6-dién-1-ol, le 3,7-diméthyloct-6-én-1-ol, le 2,6-diméthyloct-7-én-2-ol, le 3,7-diméthyloctan-1-ol, le 2-méthyl-6-méthylénoct-7-én-2-ol et le (E/Z)-3,7-diméthylnona-1,6-dién-3-ol.

2. Utilisation selon la revendication 1, pour le renforcement de la ou d'une ou de plusieurs impression(s) d'odeur agréable d'une ou de plusieurs substance(s) à odeur agréable, la/les substance(s) n'étant pas un composé de formule (I), à savoir pour le renforcement ou l'amélioration de la fraîcheur naturelle et/ou de l'éclat d'une ou de plusieurs substance(s) à odeur agréable et/ou pour le renforcement de la ou d'une note olfactive fleurie d'une ou de plusieurs substance(s) à odeur agréable, en particulier d'une note olfactive du type jasmin.

3. Utilisation selon l'une quelconque des revendications précédentes, la ou une, plusieurs ou l'ensemble des substance(s) à odeur agréable, ne correspondant pas à la formule (I), étant choisie(s) dans le groupe constitué par des substances odoriférantes dotées d'une masse molaire dans la plage de 150 g/mole à 285 g/mole, en particulier des alcools, des aldéhydes, des cétones, des éthers, des esters et des carboxylates doté(e)s d'une masse molaire dans la plage de 150 g/mole à 285 g/mole.

4. Utilisation selon l'une quelconque des revendications précédentes, le rapport de la masse totale de substances à odeur agréable, ne correspondant pas à la formule (I), à la masse totale de composé(s) de formule (I) étant supérieur ou égal à 99 : 1, de préférence supérieur à 99,9 : 0,1, particulièrement préférablement supérieur ou égal à 99,999 : 0,001, et/ou
la quantité de composé(s) de formule (I) utilisée pour le renforcement ne suffisant pas pour transmettre une odeur de musc.

5. Mélange de substances odoriférantes, de préférence huile parfumée, comprenant ou constitué par
(A) (i) un composé de formule (I) individuel ou (ii) un mélange comprenant ou constitué par deux composés de formule (I) ou plus 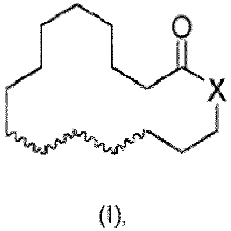
où, pour le composé de formule (I) ou pour chaque composé de formule (I)
aucune, une ou deux des quatre lignes ondulées signifie ou signifient une double liaison et les autres lignes ondulées signifient à chaque fois une simple liaison et
X est choisi parmi -O-, -CH₂- et -O-CH₂-, de préférence parmi -O- et -CH₂-, et
(B) une, deux, trois, quatre, cinq, six substances odoriférantes (supplémentaires) ou plus, la ou les substance(s) odoriférante(s) (supplémentaire(s)) n'étant pas un composé de formule (I), et une, deux, trois, quatre, cinq, six ou plus ou la totalité des substances odoriférantes (supplémentaires) possédant une masse molaire dans la plage allant de 150 g/mole à 285 g/mole, **caractérisé en ce que** le rapport de la masse totale de substances odoriférantes ne correspondant pas à la formule (I) à la masse totale du ou des composé(s) de formule (I) est supérieur ou égal à 99,9 : 0,1, particulièrement préférablement supérieur ou égal à 99,999 : 0,001,
(i) le composé de formule (I) ou (ii) un, plusieurs ou tous les composé(s) de formule (I) étant choisi(s) dans le groupe constitué par la cyclohexadéc-8-én-1-one (Aurelione, n° CAS 88642-03-9, 3100-36-5 ; Globanone, n° CAS 3100-36-5) et la cyclohexadécanone (Isomuscone, n° CAS 2550-52-9),
et la substance odoriférante ou une, plusieurs ou l'ensemble des substance(s) odoriférante(s) selon l'ingrédient (B) étant choisie(s) dans le groupe constitué par le dihydrojasmonate de méthyle, le salicylate de benzyle, le salicylate de cis-3-hexényle, le salicylate d'isoamyle, le salicylate d'hexyle, la 2,3,8,8-tétraméthyl-1,2,3,4,5,6,7,8-octa-hydro-2-naphtalénylméthylcétone, l'acétate de linalyle, l'acétate d'éthyllinalyle, le cédrylméthyléther, la cédrylméthylcétone, l'acétate de cédryle, le (4aR,5R,7aS,9R)-octahydro-2,2,5,8,8,9a-hexaméthyl-4H-4a,9-méthano-azuléno(5,6-d)-1,3-dioxole, le 1',1',5',5'-tétraméthyl-hexahydrospiro[1,3-dioxolane-2,8'(5'H)-2H-2,4a]méthanonaphtalène, le cyclododécylméthyléther, l'(éthoxyméthoxy)cyclododécane, l'acétate de décahydro-bêta-naphtyle, l'acétate de 4,7-méthano-3a,4,5,6,7,7a-hexahydro-5(-6)-indényle, le propionate d'allyl-3-cyclohexyle, le cyclohexyloxyacétate d'allyle, le benzoate de benzyle, le cinnamate de benzyle, la lactone de l'acide 15-hydroxy-pentadécanoïque, la 3-méthylcyclo-pentadécénone, le 1,3,4,6,7,8-hexahydro-4,6,6,7,8,8-hexaméthylcyclopenta[g]-2-benzopyrane, le 1-propanoate de 2-[1-(3,3-diméthylcyclohexyl)éthoxy]-2-méthyle, la 1,4-dioxacycloheptadécane-5,17-dione, la 3-méthycyclopentadécanone, le 3a,6,6,9a-tétraméthyldodécahydronaphto[2,1-b]furanne, l'alpha-irone, la bêta-irone, l'alpha-n-méthylionone, la bêta-n-méthylionone, l'alpha-isométhylionone, la bêta-isométhylionone et l'allylionone, le 2-méthyl-3-(4-tert-butylphényl)propanal, le 4-(4-hydroxy-4-méthylpentyl)-3-cyclohexènecarboxaldéhyde, le 2-méthyl-4-(2,6,6-triméthyl-1-cyclohexén-1-yl)-2-buténal, le 1-méthyl-4-(4-méthyl-3-pentén-1-yl)-3-cyclohexènecarboxaldéhyde, le 3-(3-isopropylphényl)-butyraldéhyde, le (E)-2,6,10-triméthylundéca-5,9-diénal, le benzo[1,3]dioxole-5-carbaldéhyde, le 2,2-diméthyl-3-phénylpropan-1-ol, le 2,2-diméthyl-3-m-tolylpropan-1-ol, le 1-(4-isopropylcyclohexyl)-éthanol, le (4-isopropylcyclohexyl)-méthanol, le 2-phényléthanol, le 2-isobutyl-4-méthyltétrahydropyran-4-ol, le 3,7-diméthylocta-1,6-dién-3-ol, le (Z)-3,7-diméthylocta-2,6-dién-1-ol, le (E)-3,7-diméthylocta-2,6-dién-1-ol, le 3,7-diméthyloct-6-én-1-ol, le 2,6-diméthyloct-7-én-2-ol, le 3,7-diméthyloctan-1-ol, le 2-méthyl-6-méthylénoct-7-én-2-ol et le (E/Z)-3,7-diméthylnona-1,6-dién-3-ol.

6. Mélange de substances odoriférantes selon la revendication 5, la quantité de composé(s) de formule (I) dans le mélange de substances odoriférantes étant suffisante pour renforcer la ou une ou plusieurs impression(s) d'odeur agréable de la ou d'une, de plusieurs ou de l'ensemble des substances odoriférantes selon l'ingrédient (B), en particulier pour renforcer ou pour améliorer la fraîcheur naturelle et/ou l'éclat de la ou d'une, de plusieurs ou de l'ensemble des substances odoriférantes selon l'ingrédient (B) et/ou pour renforcer la ou une note olfactive fleurie de la ou d'une, de plusieurs ou de l'ensemble des substances odoriférantes selon l'ingrédient (B), de préférence une note olfactive du type jasmin.

7. Mélange de substances odoriférantes selon l'une quelconque des revendications 5 à 6, la proportion de composé(s) de formule (I) par rapport au poids total du mélange de substances odoriférantes étant de 0,1 % en poids ou moins, particulièrement préférablement de 0,001 % en poids ou moins.

8. Produit parfumé, contenant un mélange de substances odoriférantes selon l'une quelconque des revendications 5 à 7, de préférence une huile parfumée selon l'une quelconque des revendications 5 à 7, en une quantité sensoriellement efficace, la proportion du mélange de substances odoriférantes, par rapport au poids total du produit, se situant dans la plage de 0,01 à 10 % en poids, préférablement dans la plage de 0,1 à 5 % en poids, particulièrement préférablement dans la plage de 0,25 à 3 % en poids.

9. Produit parfumé selon la revendication 8, le produit étant choisi dans le groupe constitué par les extraits de parfum, les eaux de parfum, les eaux de toilette, les lotions de rasage, les eaux de Cologne, les produits de pré-rasage, les eaux de Cologne « splash », les lingettes rafraîchissante parfumées, les produits de nettoyage acides, alcalins et neutres, les rafraîchissants textiles, les auxiliaires de repassage, les produits de lavage liquides, les produits de lavage en poudre, les agents de prétraitement de lavage, les produits assouplissants, les savons de lavage, les tablettes de lavage, les désinfectants, les désinfectants de surface, les désodorisants, les sprays aérosols, les cires et vernis, les produits de soin pour le corps, les crèmes et lotions pour les mains, les crèmes et lotions pour les pieds, les crèmes et lotions dépilatoires, les crèmes et lotions après-rasage, les crèmes et lotions bronzantes, les produits de soin des cheveux, les déodorants et les antitranspirants, les produits de cosmétique de maquillage, les bougies, les huiles pour lampes, les bâtons d'encens, les insecticides, les répulsifs et les substances combustibles.

10. Procédé de préparation d'un produit parfumé, en particulier d'un produit parfumé selon l'une quelconque des revendications 8 ou 9, comprenant les étapes suivantes :
(i) mise à disposition des ingrédients (A) et (B) tels que définis dans l'une quelconque des revendications 5 à 7, de préférence fourniture d'un mélange de substances odoriférantes selon l'une quelconque des revendications 5 à 7,
(ii) mise à disposition d'un ou de plusieurs ingrédients supplémentaires du produit parfumé à préparer, et
(iii) mise en contact ou mélange des ingrédients supplémentaires mis à disposition dans l'étape ii) avec une quantité sensoriellement efficace des ingrédients (A) et (B) mis à disposition dans l'étape i) ou du mélange de substances odoriférantes mis à disposition dans l'étape i).
